(19) Europäisches Patentamt
European Patent Office
Office européen des brevets

(11) Publication number: **0 328 830 B1**

(12) # EUROPEAN PATENT SPECIFICATION

(45) Date of publication of patent specification: **01.06.94**

(51) Int. Cl.5: **C07D 401/04**, C07D 401/14, C07D 417/14, C07D 521/00, C07D 417/04, C07D 413/14, C07D 409/14, C07D 413/04, A61K 31/445

(21) Application number: **88312149.3**

(22) Date of filing: **21.12.88**

(54) **4-Heteropentacyclic-4- N-(phenyl)amido piperidine compounds.**

(30) Priority: **31.12.87 US 139896**
**31.12.87 US 139899**

(43) Date of publication of application:
**23.08.89 Bulletin 89/34**

(45) Publication of the grant of the patent:
**01.06.94 Bulletin 94/22**

(84) Designated Contracting States:
**BE CH DE ES FR GB GR IT LI LU NL SE**

(56) References cited:
**EP-A- 0 160 422**

**CHEMICAL ABSTRACTS, vol. 65, no. 7, 26th September 1966, abstract no. 10558c-d,Columbus, Ohio, US; V. EVDOKIMOFF et al.: "Some derivatives of 4-phenyl-4-amino-1-methylpiperidine", & ANN. IST. SUPER. SANITA 1(11-12), 746-8(1965)**

(73) Proprietor: **Ohmeda Pharmaceutical Products Division Inc.**
**110 Allen Road**
**Liberty Corner, New Jersey 07938(US)**

(72) Inventor: **Lin, Bor-Sheng**
**86 Briarwood Drive**
**Berkeley Heights New Jersey 07922(US)**
Inventor: **Kudzma, Linas V.**
**7435 Boulevard East**
**North Bergen New Jersey 07047(US)**
Inventor: **Spencer, Kenneth H.**
**35 Hilltop Terrace**
**Chatham New Jersey 07928(US)**
Inventor: **Scheblein, Joseph W.**
**45 Adams Court**
**Flemington New Jersey 08822(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **Dipl.-Phys.Dr. Manitz**
**Dipl.-Ing., Dipl.-W.-Ing. Finsterwald Dipl.-Ing.**
**Grämkow Dipl.-Chem.Dr. Heyn Dipl.-Phys.**
**Rotermund**
**Morgan, B.Sc.(Phys.)**
**Postfach 22 16 11**
**D-80506 München (DE)**

## Description

The present invention relates to 4-heterocyclic-4-[(phenyl) amido] piperidine compounds and methods and compositions employing such compounds and methods of preparing the same. In particular, this new class of compounds has been found to possess desirable analgesic and anesthetic properties.

A number of patents disclose certain N-phenyl-N-(4-piperidinyl)amides having analgesic activity. For example, some such compounds are disclosed in US Patent Nos 3,164,600 and 3,998,834. US Patent No 3,164,600 discloses such compounds in which the 4 position of the piperidine ring is substituted by a lower alkyl.

EP-A-0 160 422 discloses N-aryl-N-(4-piperidinyl)amides having in 4-position of the piperidine ring a substituent selected from the group consisting of hydrogen, methoxymethyl and a carboxylate radical.

According to the report of S. McElvain et al, JACS, Vol 80 (1958), changes in the 4-position of certain substituted piperidines generally lead to less or no analgesic activity. For example, McElvain et al teaches that in going from methyl to butyl, there is no apparent effect on the degree of analgesia, and the 4-phenyl substituent fails to produce any marked effect.

According to the present invention there is provided a compound of the formula

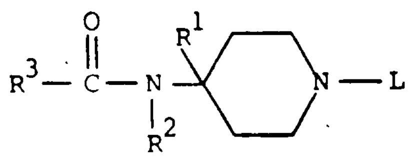

(I)

and/or a pharmaceutically acceptable acid addition salt thereof, in which formula:

$R^1$ is a thiazol-2-yl, 4-methyl-thiazol-2-yl, or 4,5-dimethylthiazol-2-yl group; a substituted 1,3,4-oxadiazolyl group wherein the substituent is a methyl, ethyl, n-propyl, n-butyl or methoxy-methyl group; a 1-methyl-imidazol-2-yl group; or 1-methyl-1 H-1,2,4-triazol-5-yl group;

$R^2$ is phenyl or 2-fluorophenyl;

$R^3$ is a methyl or ethyl group; and

L is a 2-phenylethyl, 1-phenyl-2-propyl, 2-phenyl-1-propyl, (4-ethyl-4,5-dihydro-5-oxo-1H-tetrazol-1-yl) ethyl, thiazol ethyl, benzyl, 2-(2-thienyl)ethyl, 2-(3-thienyl)ethyl, 2-(1H-pyrazol-1-yl)ethyl, 2-(2-pyridinyl)ethyl, 2-methylbutyl, ethyl-1,3,4-oxadiazolyl, 2-ethoxyethyl, 2-oxo-2-phenylethyl, 2-(4-trifluoromethyl)phenylethyl, 2-(4-fluorophenyl)ethyl, 2-(2-fluorophenyl)ethyl, 2-N-phthalimido)ethyl, 2-methylpropyl, 2-(4-methylthiazol-5-yl)-ethyl, or (3,3-diethyl-2,4-(1H,3H) pyridinedion-1-yl)ethyl group.

Compounds of the present invention are agonists and possess potent analgesic and anesthetic properties. The preferred compounds of the present invention when administered to mammals allow rapid recovery including early regain of muscle coordination. Respiratory depression during use is relatively low compared to commonly known intravenous anesthetics such as fentanyl. Heart rate decrease and arterial pressure decrease are also less. The present compounds are therefore safer, especially for coronary patients.

The compounds of the invention can exist in the form of the free base or the therapeutically or pharmaceutically acceptable acid addition salts by treatment with an appropriate acid, such as an inorganic acid, eg hydrochloric, hydrobromic, sulfuric, nitric, phosphoric acids and the like; or an organic acid such as acetic, trifluoroacetic, propionic, hydroxyacetic, methoxyacetic, benzoic, citric, oxalic, methane-sulfonic, ethanesulfonic, benzenesulfonic, toluenesulfonic, succinic, tartaric, and the like acids. Preferred acid addition salts are the chloride and oxalate or citrate. These acid addition salts can be prepared by conventional methods, eg by treatment with the appropriate acid.

Compounds of the invention having at least one asymmetric carbon atom can exist in optically active isomeric forms. For example, in compounds in which L is a 1-phenyl-2-propyl group, the carbon adjacent to the piperidinyl nitrogen is an assymetric carbon and such compounds can therefore exist in optical active isomeric (enantiomeric) forms. Such isomeric forms can be isolated from the racemic mixtures by techniques known to those skilled in the art.

The compounds of the invention, prepared as the free base, can be combined with a pharmaceutically acceptable carrier to provide a pharmaceutical composition. Suitable carriers for the free bases include propylene glycol-alcohol-water, isotonic water, sterile water for injection, USP, emulphor™-alcohol-water, cremophor-EL™ or other carriers known to those skilled in the art.

The compounds of the invention prepared as the pharmaceutically acceptable acid addition salts can also be combined with a pharmaceutically acceptable carrier to provide a pharmaceutical composition.

Suitable carriers for the acid addition salts may include an isotonic aqueous solution, or sterile water for injection, USP, alone or in combination with other solubilizing agents such as ethanol, propylene glycol, or other conventional solubilizing agents known to those skilled in the art. Of course, the carrier will vary depending upon the mode of administration desired for the pharmaceutical composition as is conventional in the art. A preferred carrier is an isotonic aqueous solution containing from 0.0001 mg/ml to 0.5 mg/ml of at least one of the compounds of this invention depending upon the pharmacology of the individual compounds being employed in the formulation.

The compounds of the invention can be administered to mammals, eg animals or humans, in amounts effective to provide the desired therapeutic effect. The compounds can be administered intravenously, intramuscularly or subcutaneously in the previously described carriers. These compounds may also be administered orally, sublingually, rectally, or transcutaneously with a suitable pharmaceutically acceptable carrier for that mode of administration as is conventional in the art.

As noted above, an effective amount of the compounds of the present invention is employed to obtain the desired therapeutic effect. Since the activity of the compounds and the depth of the desired therapeutic effect vary, the dosage level employed of the compound also varies. The actual dosage administered will be determined by such generally recognized factors as the body weight of the patient or the idiosyncrasies of the particular patient. Thus, the unit dosage for a particular patient (man) can be as low as (0.00005 mg/kg) which the practitioner may titrate to the desired effect.

The invention also provides a method of preparing a compound (I) of the formula:

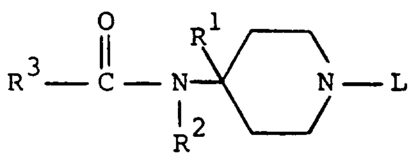

wherein

(a) a compound of formula

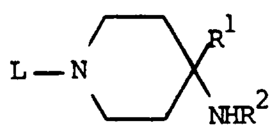

is reacted with an acylating agent containing an $R^3$ group to form said compound (I); or

(b) a compound of formula

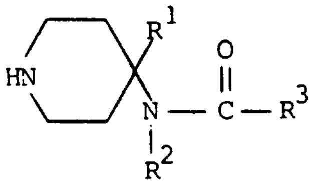

is reacted with a compound containing a L group to substitute the L group for the hydrogen bonded to the nitrogen atom of the piperidine moiety and thereby form a compound of formula (I), in which formulae:

$R^1$ is a thiazol-2-yl, 4-methyl-thiazol-2-yl, or 4,5-dimethylthiazol-2-yl group; a substituted 1,3,4-oxadiazolyl group wherein the substituent is a methyl, ethyl, n-propyl, n-butyl or methoxy-methyl group; a 1-methyl-imidazol-2-yl group; or 1-methyl-1 H-1,2,4-triazol-5-yl group;

$R^2$ is phenyl or 2-fluorophenyl;

$R^3$ is a methyl or ethyl group; and

L is a 2-phenylethyl, 1-phenyl-2-propyl, 2-phenyl-1-propyl, (4-ethyl-4,5-dihydro-5-oxo-1H-tetrazol-1-yl) ethyl, thiazol ethyl, benzyl, 2-(2-thienyl)ethyl, 2-(3-thienyl)ethyl, 2-(1H-pyrazol-1-yl)ethyl, 2-(2-pyridinyl)ethyl, 2-methylbutyl, ethyl-1,3,4-oxadiazolyl, 2-ethoxyethyl, 2-oxo-2-phenylethyl, 2-(4-trifluoromethyl)phenylethyl, 2-(4-fluorophenyl)ethyl, 2-(2-fluorophenyl)ethyl, 2-N-phthalimido)ethyl, 2-methylpropyl, 2-(4-methylthiazol-5-yl)-

ethyl, or (3,3-diethyl-2,4-(1H,3H) pyridinedion-1-yl)ethyl group.

The compounds of the present invention can be prepared beginning with known piperidones as shown below:

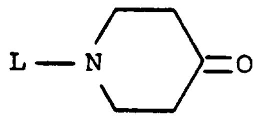

For example, the compound 1-phenylethyl-4-piperidone can be prepared according to the procedure published by A H Becket, A F Casey and G Kirk, J Med Pharm Chem, Vol 1, 37 (1959). The compound 1-benzyl-4-piperidone can be prepared in an analogous manner by the procedures described by C R Ganellin and R G Spickch, J Med Chem., Vol 8, 619 (1965) or P M Carabateas and L Grumbach, J Med Pharm Chem, Vol 5, 913(1962). Piperidones with other L groups can be prepared as disclosed in US Patent 4,584,303 and EP-A-277 794 both incorporated herein by reference.

In one example of a process of the invention, L-piperidone may be reacted with phenyl amine and the resulting Schiff base may be further reacted with, for example, a heterocyclic lithium agent to give a 4-heterocyclic-aminopiperidine or the corresponding substituted heterocyclic compound if a substituted heterocyclic amine is used. The following reaction scheme, wherein $R^1$ represents a heterocyclic group according to the present invention, illustrates such a method:

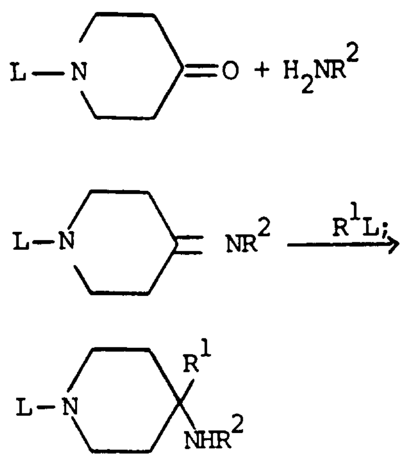


The latter compound can be reacted with the appropriate acid halide, eg $R^3$ (COCl) or anhydride $(R^3CO)_20$ to introduce the appropriate $R^3$-CO-group onto the amino nitrogen as follows.

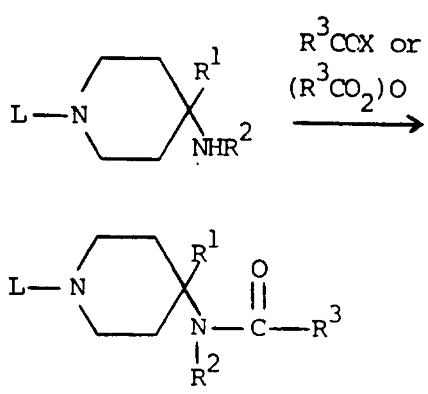


L may originally be phenylmethyl and when L is not phenylmethyl in the final product, one procedure for preparing compounds of the present invention is to subsequently split off the benzyl group and replace it

with the desired L group. For example, the compounds of the invention with the exception of sulfur bearing heterocyclic compounds may be prepared when starting with 1-(2-phenylmethyl)-4-piperidone by the following reaction scheme:

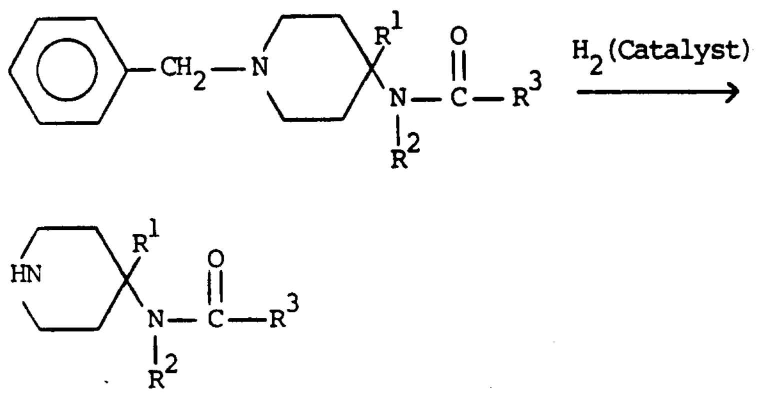


An alternative method of replacing the L group which may be used with sulfur bearing heterocyclic compounds involves employing alpha-chloroethylchloroformate to accomplish debenzylation followed by methanolysis.

The appropriate L group can then be introduced by reacting the latter compound with an appropriately reactive molecule LX wherein X is, for example, halogen such as chlorine, bromine or iodine, or a lower alkyl tosylate eg as illustrated below

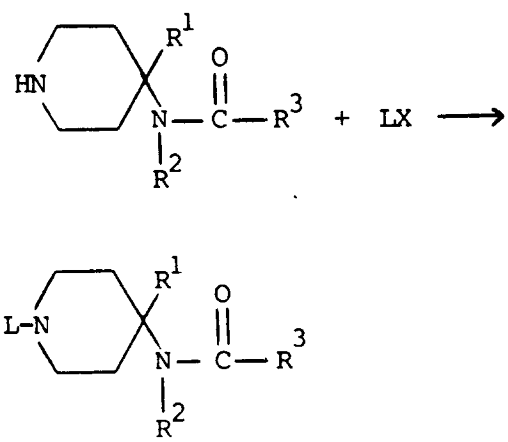


The reaction of LX can be conducted in an inert organic solvent such as, for example, N,N-dimethylformamide (DMF) or acetonitrile in the presence of an appropriate base such as alkali metal carbonate.

Compounds of the invention may also be prepared via a nitrile intermediate by the following reaction scheme:

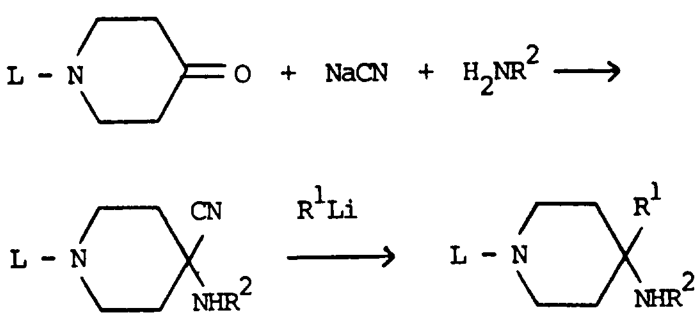

The remaining steps may proceed as shown above.

Another route to 1,3,4-oxadiazolyl compounds according to the invention is via a functional transformation of a 4-cyano substituent on the piperidine ring. Beginning with a 1-benzyl, 4-amino, 4-cyano intermediate, the addition of an azide salt results in a functional transformation to the corresponding 4-tetrazolyl compound.

Accordingly, the invention also provides a method of preparing an intermediate (IV) of formula

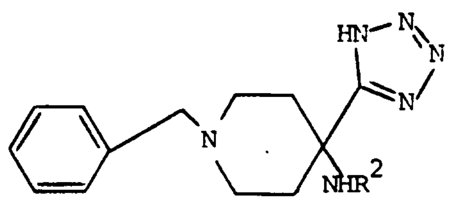

by converting a cyano group in a compound of formula:

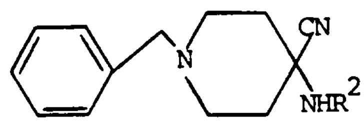

to a tetrazolyl group, wherein $R^2$ is as defined above. The reaction is preferably performed in a non-aqueous reaction medium such as tetrahydrofuran with an azide salt, preferably sodium azide, in the presence of a Lewis acid such as aluminium chloride.

The intermediate (IV) provides a particularly useful route to 1,3,4-oxadiazolyl compounds according to the invention.

1,3,4-oxadiazolyl compounds may be formed by acylating the intermediate (IV) with an acylating agent so as to convert the tetrazolyl group to an $R^3$ substituted 1,3,4-oxadiazolyl group while simultaneously acylating the $R^2NH_2$ group to convert it to an $R^2NCOR^3$ group. The acylating agent may be an acid anhydride or acid halide, and the reaction may be conducted at a temperature of 110 to 160 °C. The benzyl group may then be removed by catalytic hydrogenation and a different L group may then be substituted for the hydrogen bonded to the nitrogen atom of the piperidine ring by reaction with a compound of formula LX where X is a halogen radical in a reaction medium typically comprising sodium carbonate in methyl nitrile so as to form an analgesic or anaesthetic compound according to the invention.

If the substituent on the 1,3,4-oxadiazolyl ring is required to be different from the $R^3$ group, the acylation of the $R^2NH_2$ group may be performed first by reaction with an acylating agent including the $R^3$ group under relatively mild conditions (eg at a temperature in the range 70 to 75 °C) which leave the tetrazolyl group unchanged, and then the resulting product can be acylated with an acylating agent including an $R^4$ group under more severe conditions (eg at a temperature in the range 110 to 160 °C) so as to convert the tetrazolyl group to an $R^4$ substituted 1,3,4-oxadiazolyl group wherein $R^4$ is methyl, ethyl, n-propyl, n-butyl or methoxymethyl and different from $R^3$.

A different L group may then be substituted for the benzyl group bonded to the nitrogen atom of the piperidine ring by procedures analogous to the ones described above.

These reactions involving the intermediate (IV) are illustrated below.

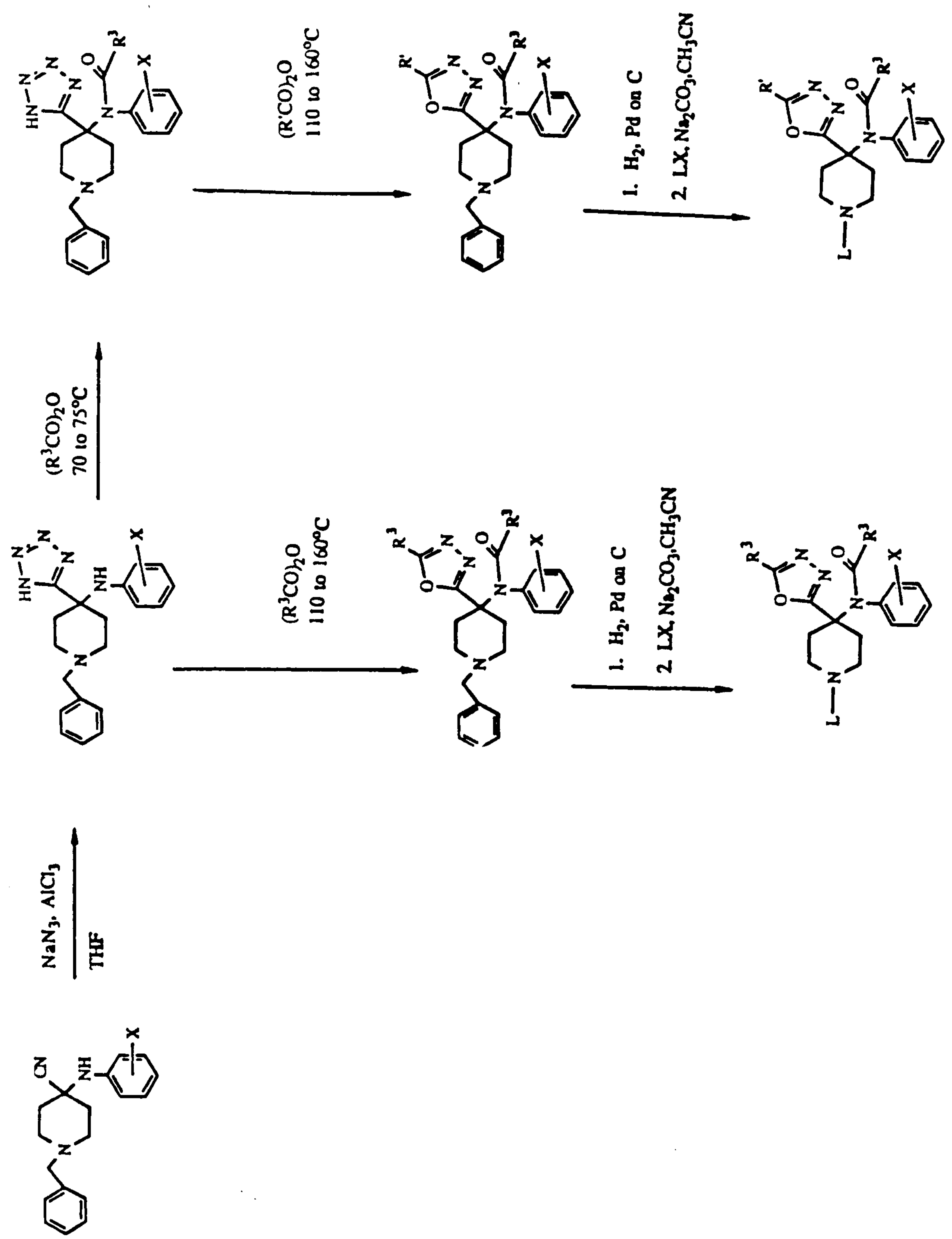


The following Examples A to H refer to the preparation of intermediates and Examples 1 to 13 refer to the preparation of compounds according to the present invention.

Example A:

In a well ventilated hood a solution of potassium cyanide (110.5 gms, 1.69 mol) in 680 mls of deionized water was stirred at room temperature (RT). To this solution was added aniline (157.6 gms, 1.69 mol) in 320

mls of methanol. The resulting solution was cooled in an ice/water bath and 12N HCl (140 mls, 1.68 mol) was added dropwise with cooling (Caution HCN). N-benzyl-4-piperidone (320 gms, 1.69 mol) was slowly added with cooling followed by warming reaction mixture to RT. The reaction mixture was stirred at RT for 8 days. When stirring was stopped the reaction mixture separated to two layers. The top aqueous methanol layer was decanted to leave a gummy solid. 200 mls of isopropanol was added to the gummy residue and the mixture was vigorously stirred for 45 minutes. A finely divided solid was formed. This solid was filtered, washed with isopropanol and dried in an over (50°C) to give 288.2 gms (58.5%) of the desired alpha-amino nitrile as a white powder.

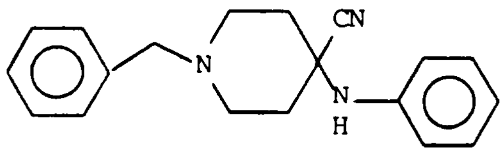

Example B:

1-benzyl-4-(4-methyl-thiazol-2-yl)-4-(N-phenyl)piperidine

A solution of 2.5M butyllithium in hexane (103 mls, 257.5 mmol, Aldrich) was added to 150 mls of dry THF under argon and the solution was cooled to -78°C. 4-Methyl-thiazole (25 mls, 274 mmol, Aldrich 98%) was added to the cold butyllithium solution dropwise via syringe. The solution turned a deep orange and after 5 min. at -78°C a solution of nitrile (38.54 gms, 132 mmol) prepared in Example 1 dissolved in 350 mls of THF was added rapidly via a cannula. The reaction was stirred at -78°C for 5 min. followed by warming to 0°C in a water/ice bath. The reaction was quenched at 0°C by slow dropwise addition of 50 mls of water and stirred for 0.5 hrs. The organic layer was separated, dried over $Na_2SO_4$ and concentrated to give the crude diamine as a dark oil. The oil passed through a column of silica 60 (230-400 mesh) eluting with 1:2 EtOAc/Hexane and the resulting pale yellow oil crystallized from hot hexane to give 40.4 gms (84%) of pore diamine.

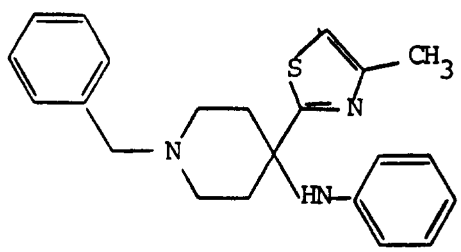

NMR: 7.50-6.40 (m,11H), 4.25 (br s,1H), 3.50(s,2H), 2.45 (s,3H), 2.90-1.90 (complex,8H)

Example 1:

1-benzyl-4-(4-methyl-thiazol-2-yl)-4-(N-phenylpropionamido))piperidine

The diamine (6.97 gms, 19.17 mmol) of Example 2 was dissolved in 150 mls. of dry chloroform and an excess of propionyl chloride (10 mls) was added. The reaction mixture has heated at reflux for 5 days, after which no starting diamine could be detected by TLC. The reaction was cooled and added dropwise to a cold (0°C) solution of KOH (25 gms) in 500 mls of water and stirred for 2 hrs. The organic layer was separated, dried over $Na_2SO_4$ and concentrated in vacuo to give the desired amide as an amber glass (7.63 gms, 95%).

NMR: 7.50 (s,5H), 7.35 (s,5H), 6.85 (s,1H), 3.40 (s,2H), 2.85-1.50 (complex,10H), 2.40 (s,3H), 0.80 (t,3H)

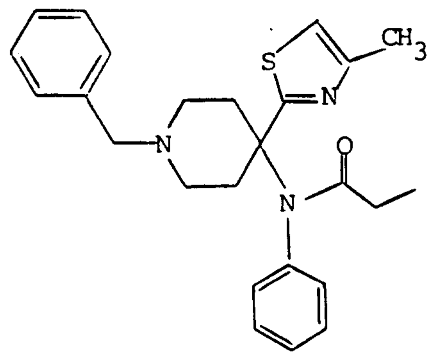

Example C:

The amide (9.78 gms, 22.35 mmol) of Example 3 was dissolved in 250 mls of 1,2-dichloroethane and cooled to 0°C in an ice bath followed by addition of 1-chloroethyl chloroformate (3.53 gms, 24.69 mmol). The solution was warmed to RT and heated to reflux. After 2.5 hrs at reflux the dichloromethane was stripped off in vacuo and the orange residue was redissolved in 200 mls of methanol. The methanol solution was refluxed for 5 hrs, cooled and concentrated in vacuo. The residue was taken up in 400 mls 0.5N HCL and extracted twice with 200 ml portions of ethyl ether. The aqueous layer was basified with 25% aqueous NaOH and extracted with chloroform. The chloroform layer was separated, dried over $Na_2SO_4$ and concentrated to give nor-compound (7.48 gms, 96%) as an oil.

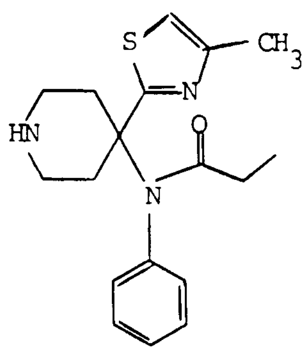

Example 2:

The nor-compound (1.06 gms, 3.22 mmol) of Example 4 was dissolved in 40 mls of acetonitrile. The solution was stirred and $K_2CO_3$ (1.8 gms) and phenethyl bromide (0.90 gms, 4.86 mmol) were added. The reaction was heated at reflux for 2 days after which the reaction was cooled filtered and concentrated in vacuo. The residue was chromatographed on silica 60 (230-400 mesh) eluting with 1:1 EtOAc/Hex to give compound as a pale yellow oil (1.33 gms, 95%).

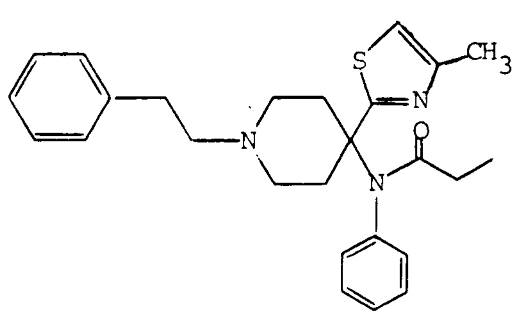

Example 3:

1-[2-(1H-pyrazol-1-yl)ethyl]-4-(4-methyl-thiazol-2-yl)-4-(N-phenylpropionamido) piperidine

The nor-compound (1.06 gms, 3.2 mmol) of Example 4 was dissolved in 45 mls of acetonitrile. The solution was stirred and $K_2CO_3$ (1.35 gms) and 2-(1H-pyrazol-1-yl) ethyl tosylate (0.93 gms, 3.4 mmol) were added. (For the preparation of 2-(1H-pyrazol-1-yl)ethyl tosylate see Machine P J et al, Journal of Medicinal Chemistry, Volume 27, pp 503 to 509, 1984.) The reaction was heated at reflux for 3 days after which the reaction was cooled filtered and concentrated in vacuo. The residue was chomatographed on silica 60 (230-400 mesh) eluting with 5% methanol in ethyl acetate to give the desired product as an amber oil (1.21 gms, 92%)

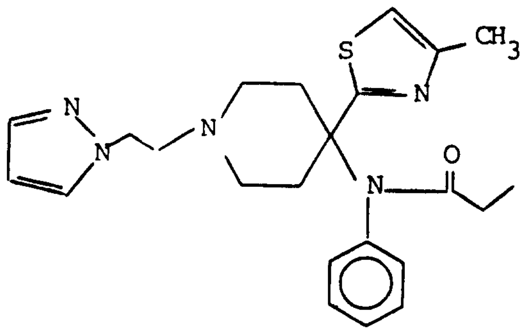

| %CHN Analysis of oxalate salt mp = 185-87 ° C | | | |
|---|---|---|---|
| Calc. | %C(58.46) | %H(6.08) | %N(13.64) |
| Found | 58.19 | 6.05 | 13.46 |

NMR: 7.35 (s,5H), 6.90 (s,1H), 6.25(m,1H), 4.25 (m,2H), 2.90-2.10 (complex,13H), 1.90 (q,2H), 0.90 (t,3H)

Example D:

[1-benzyl-4-(1-methyl-1,2,4-triazol-5-yl)-4-anilino- piperidine]

A 500 ml rb flask was charged with N-benzylpiperidone (22.72 g, 120 mmol), aniline (11.17 g, 120 mmol), p-toluene-sulfonic acid (200 mg) and toluene (250 ml). The mixture was refluxed for 3 hours while separating water through a Dean-Stark trap. The resulting reddish brown solution was cooled to 0 ° C.

A 1000 ml rb flask was charged with 1-methyl-1,2,4-triazole (13.28 g, 160 mmol) and dry THF (400 ml). To this THF solution at -78 ° C under $N_2$, n-BuLi (100 ml, 1.6 M in Hex) was added. The resulting milky mixture was kept stirring at -78 ° C for 2 hours. To this lithio mixture at -78 ° C, the above toluene solution was added through a needle. After the addition, the resulting mixture was stirred at -78 ° C for 30 minutes, gradually warmed up to room temperature and stirred at room temperature for 30 minutes. The reaction was quenched with $H_2O$ (300 ml). The organic layer was separated, dried over $Na_2SO_4$ and concentrated in vacuo. The resulting residue was chromatographed to give the product (20.53 g, 59 mmol) as an amber viscous oil: IR (neat) 3400 $cm^{-1}$; NMR (60MHz, $CDCl_3$) 3.53 (s,2H, benzyl $CH_2$), 3.93 (s,3H, triazolyl $CH_3$), 6.2 to 7.5 (m's, 10H, phenyl H's), 7.86 (s, 1H, triazolyl H).

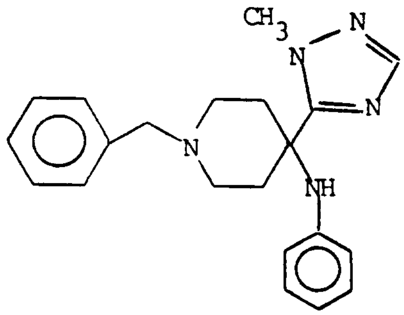

Example 4:

1-benzyl-4-(1-methyl,1,2,4-triazol-5-yl)-4-(N-phenyl- propionamido) piperidine

A mixture of 1-benzyl-4-(1-methyl-1,2,4-triazol-5-yl)-4-anilinopiperidine (11.45 g, 33 mmol) and propionic anhydride (40 ml) was stirred at 140 °C for 70 hrs. It was then concentrated in vacuo. The crude residue was dissolved in $CH_2Cl_2$, washed with 5% $Na_2CO_3$, dried over $Na_2SO_4$ and concentrated in vacuo. The resulting residue was chromatographed ($SiO_2$ EtOAc) to yield the product (8.59 g, 21 mmol); as an amber viscous oil: IR (neat) 1660 $cm^{-1}$; NMR (60 MHZ, $CDCl_3$) 0.86 (t, 3H, $\underline{C}H_3CH_2CON$), 1.5-3.0 (complex, 10H), 3.35 (s, 2H, benzyl $CH_2$), 4.23 (s, 3H, triazolyl $CH_3$), 7.23 and 7.40 (2bs, 10H, phenyl H's), 7.83 (s, 1H, triazolyl H).

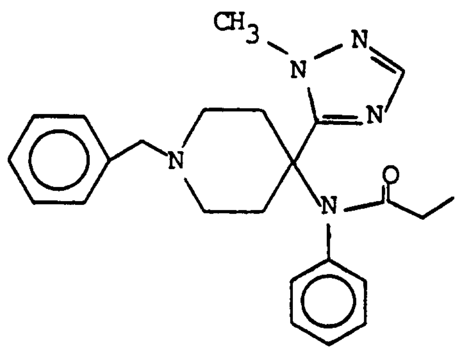

Example E:

1-benzyl-4-(1H-tetrazol-5-yl)-4- anilinopiperidine

Sodium azide (5.85 g, 90 mmol) and 1-benzyl-4-cyano- 4-anilinopiperidine (2.91 g, 10 mmol) were added slowly to a solution of $AlCl_3$ (2.67 g, 20 mmole) in dry THF (100 ml). The resulting mixture was refluxed overnight. The reaction was quenched with water (50 ml). After separating the THF layer, the aqueous layer was extracted with additional THF (4 x 50 ml). The combined THF layers were dried over $MgSO_4$ and concentrated in vacuo. The solid obtained was dissolved in 1N.HCl solution and the pH was adjusted to 6.0 with 1N NaOH solution. The resulting mixture was left overnight and filtered to give the product (1.58 g, 4.7 mmol) as white crystalline solid: mp 205-206 °C (decomposed); IR (nujol) 3300 $cm^{-1}$; NMR (270 MHz, DMSO-$d_6$) 2.2 to 2.7 (m, 8H, piperidine $CH_2$), 3.53 (s, 2H, benzyl $CH_2$), 6.35, 6.63 and 7.01 (3m, 5H, anilinophenyl H's), 7.25 (m, 5H, benzylphenyl H's). Anal Calcd for $C_{19}H_{22}N_6$: C, 68.24; H,6.63; N,25.13. Found: C, 68.79; H,6.76; N,24.81.

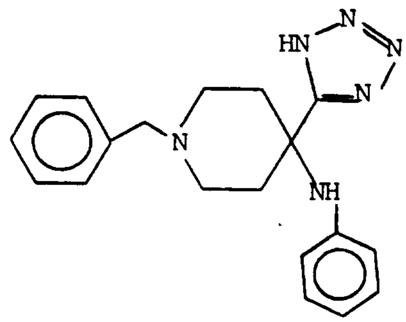

Example F:

1-benzyl-4-(1H-tetrazol-5-yl)-4-(2-fluoroanilino)-piperidine

To 250 ml of stirring dry THF in a 500 ml rb flask at 0°C under $N_2$, $AlCl_3$ (23.0 g, 172 mmol) was added in small portions. Subsequently, $NaN_3$ (47.4 g, 729 mmol) and 1-benzyl-4-cyano-4-(2-fluoroanilino)-piperidine (21.8 g, 70 mmol) were added. The resulting mixture was refluxed for 20 hours. It was cooled to room temperature and poured onto stirring water (300 ml). The THF layer was separated and the aqueous layer was extracted with additional THF (3 x 500 ml). The combined THF layers were dried over $MgSO_4$ and filtered. The filtrate was kept at 0°C for 15 hours. After a filtration, the desired compound (17.1 g, 49 mmol) as white powder was obtained: m.p. 195-198°C; IR (nujol) 3360 $cm^{-1}$; NMR (270 MHz, DMSO-$d_6$) 2.29, 2.46 and 2.67 (3m, 8H, piperidine $CH_2$'s), 3.53 (s, 2H, benzyl $CH_2$), 6.12, 6.61, 6.72 and 6.98 (4m, 4H, anilinophenyl H's), 7.2-7.4 (m, 5H, benzylphenyl H's). Anal Calcd for $C_{19}H_{21}FN_6$: C,64.75; H,6.01; N,23.85. Found: C, 64.52; H,5.94; N,23.56.

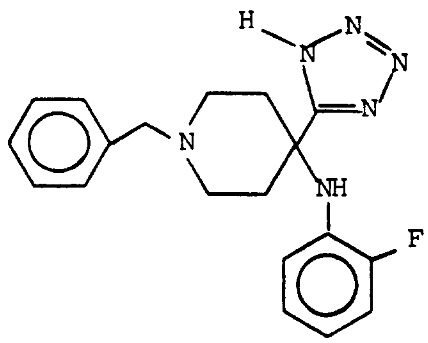

Example G:

1-benzyl-4-(1H-tetrazol-5-yl)-4-(N-phenylpropionamido)-piperidinium propionate

A 250 ml rb flask was charged with 1-benzyl-4- (1H-tetrazol-5-yl)-4-anilinopiperidine (17.7 g, 53 mmol) and propionic anhydride (95 ml). The resulting mixture was stirred at 70°-75°C for 6 hours. Then, it was concentrated in vacuo and the crude was chromatographed ($SiO_2$, 5-20% MeOH/$CH_2Cl_2$) to give the desired compound (14.7 g, 32 mmol) as white powder: m.p. 226-228°C; IR (nujol) 1660 $cm^{-1}$; NMR (270 MHz, $CDCl_3$) 0.864, (t, 3H, $\underline{CH_3}CH_2CON$), 1.175 (t, 3H, $\underline{CH_3}CH_2$ $CO_2H$), 1.883 (g, 2H, $CH_3\underline{CH_2}CO_2H$), 2.113 (m, 4H), 2.395 (g, 2H, $CH_3\underline{CH_2}CO_2H$), 2.8-3.1 (m,4H), 3.464 (s, 2H, benzyl $CH_2$), 7.1 to 7.5 (complex, 10H, phenyl H's). Anal. Calcd. for $C_{22}H_{26}N_6O$. $C_3H_6O_2$: C,64.64; H,6.94; N,18.09. Found: C, 64.65; H,7.00; N,17.89.

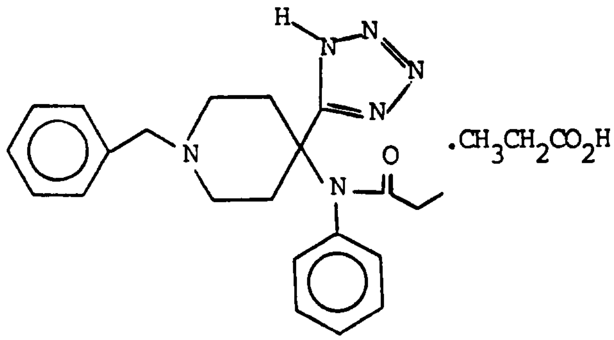

Example 5:

1-benzyl-4-(methyl-1,3,4-oxadiazolyl)-4-(N-phenylacetamido) piperidine

A 25 ml rb flask was charged with 1-benzyl-4-(1H-tetrazol-5-yl) -4-anilinopiperidine (592 mg, 1.77 mmol) and acetic anhydride (10 ml). The resulting mixture was refluxed for 2 hours and concentrated in vacuo. The resulting residue was dissolved in $CH_2Cl_2$ (50 ml), washed with 5% $NaHCO_3$ and $H_2O$. It was dried over $Na_2SO_4$ and concentrated in vacuo. The residue was chromatographed ($SiO_2$, EtOAc) to afford the product (525 mg, 1.34 mmol) as a colorless liquid: IR (neat) 1660 $cm^{-1}$; NMR (60 $MH_z$, $CDCl_3$) 1.67 (s, 3H, $CH_3CO$), 2.60 (s, 3H, oxadiazolyl $CH_3$), 3.44 (s, 2H, benzyl $CH_2$), 7.30 and 7.48 (2bs, 10H, phenyl H's). Anal Calcd for $C_{23}H_{26}N_4O_2.C_2H_2O_4$: C,62.49; H,5.87; N,11.66. Found: C, 62.56; H,5.95; N,11.37.

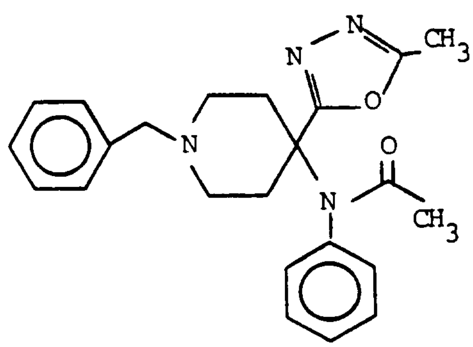

Example 6:

1-benzyl-4-(ethyl-1,3,4-oxadiazolyl)-4-(N-phenylpropionamido) piperidine

A mixture of 1-benzyl-4-(1H-tetrazol-5-yl)-4-anilino-piperidine (7.45 g, 22 mmol) and propionic anhydride (100 ml) was stirred at 120 °C for 2 hours ($N_2$ evolution lasted about 20 minutes). The resulting solution was concentrated in vacuo. The crude was dissolved in $CH_2Cl_2$ (400 ml), washed with 5% $NaHCO_3$ (200 ml) , dried over $Na_2SO_4$ and concentrated in vacuo. The residue was chromatographed ($SiO_2$, EtOAc/Hex 1:1) to yield the product (8.20 g, 19.6 mmol) as an amber oil: IR (neat) 1670 $cm^{-1}$; NMR (60 MHz, $CDCl_3$) 0.89 (t, 3H, $CH_3CH_2CO$), 1.39 (t, 3H, oxadiazolyl $CH_3$), 3.34 (s, benzyl $CH_2$), 7.30 and 7.49 (2bs, 10H, phenyl H's). Anal Calcd for $C_{25}H_{30}N_4O_2.C_2H_2O_4$: C,63.76; H,6.34; N,11.02. Found: C, 63.40; H,6.26; N,10.79.

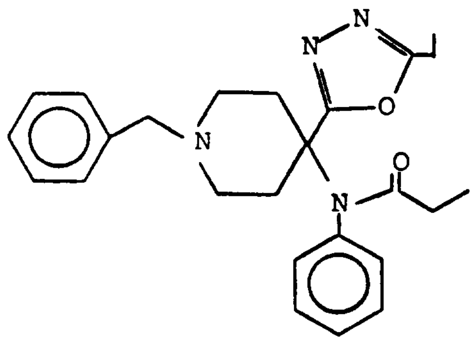

Example 7:

1-benzyl-4-(methyl-1,3,4-oxadiazolyl)-4-(N-phenylpropionamido) piperidine

A 100 ml rb flask was charged with 1-benzyl-4-(1H-tetrazol-5-yl)-4-(N-phenylpropionamido)piperidinium propionate (6.04 g, 13 mmol) and acetic anhydride (50 ml). The resulting mixture was stirred at 110 °C for 2 hours and concentrated in vacuo. The crude residue was dissolved in $CH_2Cl_2$ (100 ml), washed with 5% $Na_2CO_3$ (100 ml), dried over $Na_2SO_4$ and concentrated in vacuo. The resulting residue was chromatographed ($SiO_2$, EtOAc/Hexane 1:1) to give the product (4.20 g, 10.4 mmol) as a viscous oil: NMR (60 MHz, $CDCl_3$) 0.90 (t, 3H, $\underline{CH_3}CH_2CO$), 2.61 (s, 3H, oxadiazolyl $CH_3$), 3.46 (s, 2H, benzyl $CH_2$), 7.33 and 7.52 (2bs, 10H, phenyl H's).

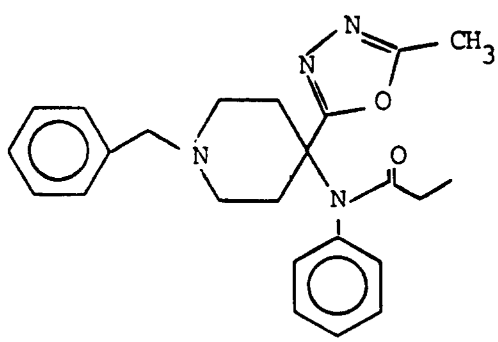

Example 8:

1-benzyl-4-methoxymethyl-1,3,4-oxadiazolyl)-4-(N-phenyl-propionamido) piperidine

To a solution of methoxyacetic acid (4.51 g, 50 mmol) in tripropylamine (80 ml) at 0 °C, methoxyacetyl chloride (5.80 g, 53 mmol) was added dropwise. The resulting solution was stirred at 0 °C for 15 minutes. Subsequently, 1-benzyl-4-(1H-tetrazol-5-yl)-4-(N-phenylpropionamido) piperidinium propionate (4.42 g, 9.5 mmol) was added. The whole mixture was stirred at 110 °C for 3 hours and then concentrated in vacuo. The crude was dissolved in ethyl acetate (300 ml) , washed with 5% $Na_2CO_3$ (200 ml), dried over $Na_2SO_4$ and concentrated in vacuo. The resulting residue was chromatographed ($SiO_2$, EtOAc/Hexane 1:1) to give the product (4.15g, 9.5 mmol) as an amber oil: NMR (60MHz, $CDCl_3$) 0.89 (t, 3H, $\underline{CH_3}CH_2CO$), 3.46 (bs, 5H, $\underline{CH_3}OCH_2$ and $Ph\underline{CH_2}$), 4.70 (s, 2H, $CH_3O\underline{CH_2}$), 7.27 and 7.47 (2bs, 10H, phenyl H's).

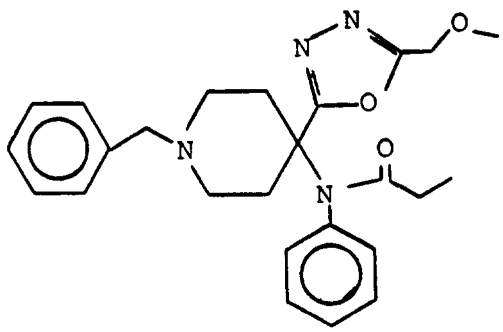

Example 9 :

1-benzyl-4-(ethyl-1,3,4-oxadiazolyl)-4-[N-(2-fluorophenyl) propionamido] piperidine

A 250 ml rb flask was charged with 1-benzyl-4-(1H-tetrazol-5-yl)-4-(2-fluoroanilino)piperidine (12.0 g, 34 mmol) and propionic anhydride (80 ml). The resulting mixture was stirred at 160 °C for 48 hours and then concentrated in vacuo. The crude was dissolved in $CH_2Cl_2$ (200 ml), washed with 5% $Na_2CO_3$ (100 ml), dried over $Na_2SO_4$ and concentrated in vacuo.

The resulting residue was chromatographed ($SiO_2$, EtOAc/Hexane 2:1) to yield the product (5.75 g, 13 mmol) as a viscous oil: NMR (60 MHz, $CDCl_3$) 0.93 (t, 3H, $\underline{CH_3}CH_2CO$), 1.40 (t, 3H, oxadiazolyl $CH_3$), 3.50 (s, 2H, benzyl $CH_2$), 7.1-7.8 (m, 9H, phenyl H's).

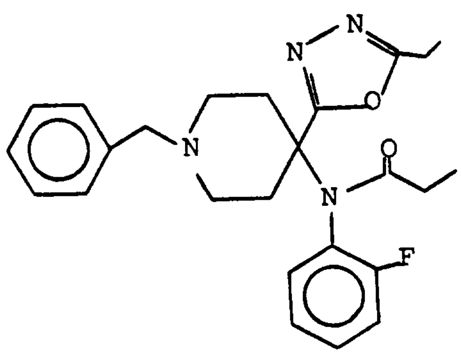

Example H:

4-(ethyl- 1,3,4-oxadiazolyl)-4-(N-phenylpropionamido) piperidine

A mixture of 1-benzyl-4-(ethyl-1,3,4-oxadiazolyl)-4-(N-phenylpropionamido) piperidine (8.20 g, 19.6 mmol) and 10% palladium on charcol (2.0 g) in methanol (100 ml) was reacted with hydrogen (50 psi) in a Parr shaker. After 24 hours, the resulting mixture was filtered and the filtrate was concentrated in vacuo. A foamy residue (6.42 g, 19.5 mmol) was obtained. It was used for reactions without further purification.

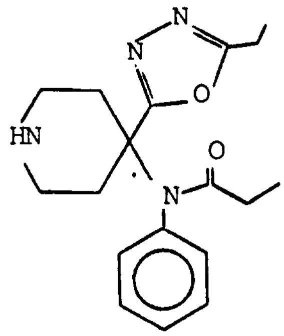

Example 10:

1-(2-phenylethyl)-4-(ethyl-1,3,4-oxadiazolyl)-4-(N-phenyl-propionamido) piperidine

A mixture of 4-(ethyl-1,3,4-oxadiazolyl)-4-(N-phenyl-propionamido) piperidine (900 mg, 2.74 mmol), phenethyl bromide (507 mg, 2.74 mmol), NaI (490 mg, 3.3 mmol), $Na_2CO_3$ (2.0 g) in acetonitrile (50 ml) was refluxed for 72 hours. It was filtered and concentrated in vacuo. The residue was treated with $CH_2Cl_2$ - (100 ml) and filtered again. The filtrate was concentrated in vacuo and the resulting residue was chromatographed ($SiO_2$, EtOAc/Hex are 1:1) to give the product (904 mg, 2.1 mmol) as a colorless oil: IR (neat) 1660 $cm^{-1}$; NMR (60 MHz, $CDCl_3$) 0.87 (t, 3H, $C\underline{H}_3CH_2CON$), 1.37 (t, 3H, oxadiazolyl $CH_3$), 1.60 - 3.15 (m, 16H), 7.23 and 7.50 (2bs, 10H, phenyl H's).

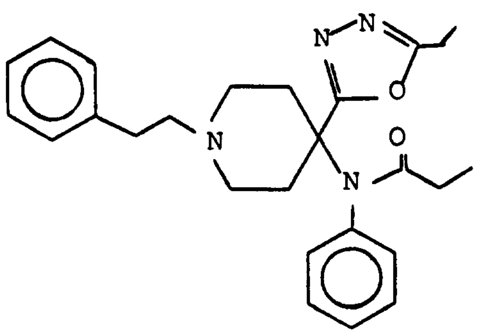

Example 11:

1-(2-phenethyl)-4-(ethyl-1,3,4-oxadiazolyl)-4-(N-phenyl-propionamido) piperidinium oxalate

To a solution of 1-(2-phenylethyl)-4-(ethyl-1,3,4-oxadiazolyl)-4-(N-phenylpropionamido)piperidine (417 mg, 0.96 mmol) in ethyl acetate (20 ml), oxalic acid (87 mg, 0.97 mmol in 2 ml EtoAc) was added dropwise. The resulting mixture was heated to 70°C and methanol added dropwise until it became a clear solution. The solution was cooled to room temperature on standing for 2 hours. After filtration, the product (408 mg, 0.77 mmol) as a crystalline compound was obtained, mp 159-161°C. Anal Calcd for $C_{26}H_{32}N_4O_2$; $C_2H_2O_4$; 1/4 $H_2O$: C,63.80; H,6.60; N,10.63.
Found: C,63.93; H,6.65; N,10.75.

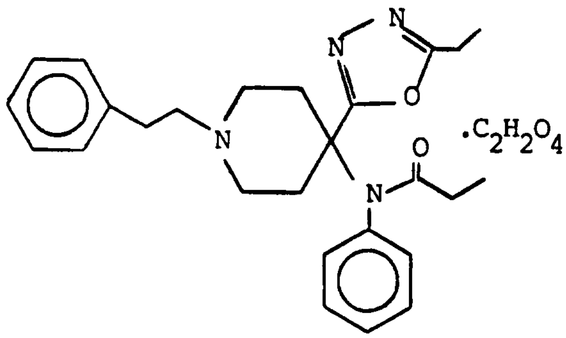

Example 12:

1-[2-(2-thienyl)ethyl]-4-(ethyl-1,3,4-oxadiazolyl)-4-(N- phenyl-propionamido) piperidine

A mixture of 4-(ethyl-1,3,4-oxadiazolyl)-4-(N-phenyl propionamido)piperidine (880 mg, 2.68 mmol), 2-(2-thienyl) ethyl chloride (385 mg, 2.63 mmol) (see Example XI of EP-A 160 422 for the preparation of this compound), NaI (470 mg, 3.1 mmol) and $Na_2CO_3$ (2.0 g) in acetonitrile (50 ml) was refluxed for 72 hours. It was cooled down to room temperature and filtered. The filtrate was concentrated in vacuo and the residue was treated with $CH_2Cl_2$ (100 ml). The resulting mixture was filtered and the filtrate was concentrated in vacuo. The residue was chromatographed to give the product (940 mg, 2.14 mmol) as an amber oil: IR (neat) 1660 $cm^{-1}$; NMR (60MHz, $CDCl_3$) 0.88 (t, 3H, $\underline{CH_3}CH_2CO$), 1.39 (t, 3H, oxadiazolyl $\underline{CH_3}$), 1.60 to 3.20 (m, 16H), 6.70 to 7.40 (m, 3H, thienyl H's) 7.44 (s, 5H, phenyl H's).

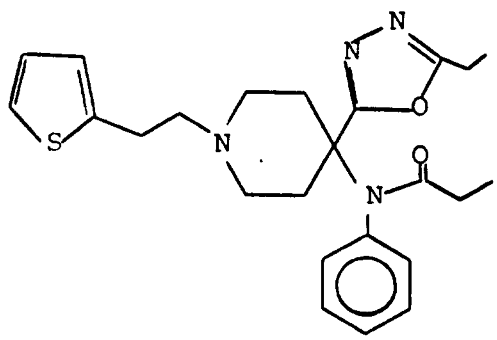

Example 13:

1- [2-(2-thienyl)ethyl)-4-(ethyl-1,3,4-oxadiazolyl)-4-(N-phenyl-propionamido) piperidinium oxalate

To a solution of 1-[2-(2-thienyl)ethyl]-4-(ethyl-1,3,4-oxadiazolyl)-4-(N-phenylpropionamido) piperidine (660 mg, 1.50 mmol) in ethyl acetate (20 ml), oxalic acid (136 mg, 1.51 mmol, in 3 ml EtoAc) was added dropwise. The resulting mixture was heated to 70 °C and methanol added dropwise until it became a clear solution. The solution was cooled to room temperature on standing for 2 hours. The precipitate was filtered to afford the product (630 mg, 1.19 mmol) as a white crystalline compound, mp 160-162 °C. Anal Calcd for $C_{24}H_{30}N_4O_2S$ $C_2H_2O_4$: C,59.07; H,6.10; H,10.60 Found: C,59.08; H,6.26; N,10.45.

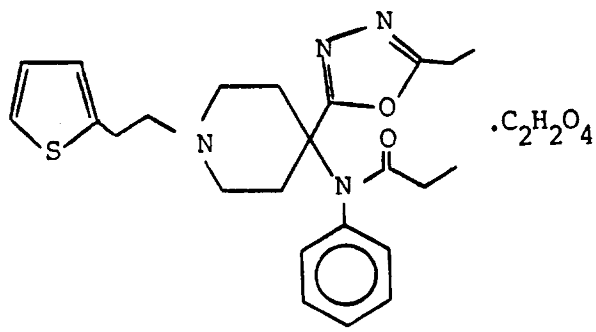

A number of compounds in accordance with the present invention were tested for their analgesic and reversal properties. Specifically, the acid addition oxalate salts of the compounds tested in accordance with the invention were dissolved in sterile water for injection, USP, to form a solution whose concentration varied from 0.00001 mg/ml to 5 mg/ml. The solution was administered intravenously in a mouse tail vain.

The $ED_{50}$ values were obtained from the mouse hot plate analgesia test (58 °C) described in Domer, Floyd R, Animal Experiments in Pharmacological Analysis, Charles C Thomas, Springfield, 1971, p 283 ff. The compounds listed in Table 1 below were tested by this procedure and found to have the analgesic activities listed in Tables I, II and III below.

Table I

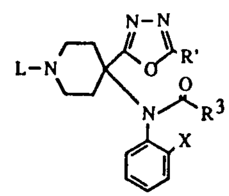

| Compound | L | R' | $R^3$ | X | M.P. (°C) | $ED_{50}$mg/kg mouse hot plate |
|---|---|---|---|---|---|---|
| 1-benzyl-4-(methyl-1,3,4-oxadiazolyl)-4-(N-phenylacetamido)piperidinium oxalate | 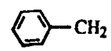 | $CH_3$ | $CH_3$ | H | 198-200 | >5.0 |
| 1-(2-phenylethyl)-4-(methyl-1,3,4-oxadiazolyl)-4-(N-phenylacetamido)piperidinium oxalate | 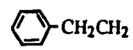 | $CH_3$ | $CH_3$ | H | 219-221 | 0.395 |
| 1-[2-(thienyl)ethyl)-4-(methyl-1,3,4-oxadiazolyl)-4-(N-phenylacetamido)piperidinium oxalate | 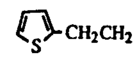 | $CH_3$ | $CH_3$ | H | 237-239 | >1.0 |
| 1-[2-(4-ethyl-4,5-dihydro-5-oxo-1H-tetrazol-1-yl)ethyl]-4-(methyl-1,3,4-oxadiazolyl)-4-(N-phenylacetamido)piperidinium oxalate | 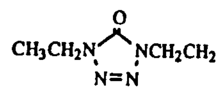 | $CH_3$ | $CH_3$ | H | 174-176 | >5.0 |
| 1-[2-(1-pyrazolyl)ethyl]-4-(methyl-1,3,4-oxadiazolyl)-4-[N-(2-fluorophenyl)acetamido]piperidinium chloride | 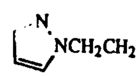 | $CH_3$ | $CH_3$ | F | 131-135 | >5.0 |
| 1-[2-(2-thienyl)ethyl]-4-(methyl-1,3,4-oxadiazolyl)-4-[N-(2-fluorophenyl)acetamido]piperidinium chloride | 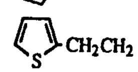 | $CH_3$ | $CH_3$ | F | 155-160 | 0.156 |
| 1-[2-(3-thienyl)ethyl]-4-(methyl-1,3,4-oxadiazolyl)-4-[N-(2-fluorophenyl)acetamido]piperidinium chloride | 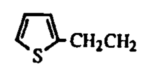 | $CH_3$ | $CH_3$ | F | 143-147 | 0.159 |
| 1-(2-methylbutyl)-4-(methyl-1,3,4-oxadiazoyl)-4-[N-(2-fluorophenyl)acetamido]piperidinium chloride | $CH_3CH_2CHCH_3CH_2$ | $CH_3$ | $CH_3$ | F | 126-128 | 0.458 |

Table I...continued

| Compound | L | R' | $R^3$ | X | M.P. (°C) | $ED_{50}$mg/kg mouse hot plate |
|---|---|---|---|---|---|---|
| 1-(2-phenylethyl)-4-(methyl-1,3,4-oxadiazolyl)-4-[N-(2-fluorophenyl)acetamido]piperidinium chloride | 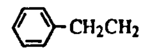 | $CH_3$ | $CH_3$ | F | 150-155 | 0.059 |
| 1-[2-(4-ethyl-4,5-dihydro-5-oxo-1H-tetrazol-1-yl)-ethyl]-4(methyl-1,3,4-oxadiazolyl)-4[N-(2-fluorophenyl)-acetamido]piperidinium chloride | 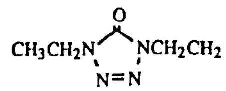 | $CH_3$ | $CH_3$ | F | 133-137 | >5.0 |
| 1-benzyl-4-(ethyl-1,3,4-oxadiazolyl)-4-(N-phenyl-propionamido)piperidinium oxalate | 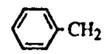 | $CH_3CH_2$ | $CH_3CH_2$ | H | 180-182 | >5.0 |
| 1-(2-phenylethyl)-4-(ethyl-1,3,4-oxadiazolyl)-4-(N-phenyl-propionamido)piperidinium oxalate | 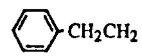 | $CH_3CH_2$ | $CH_3CH_2$ | H | 159-161 | 0.215 |
| 1-[2-(2-thienyl)ethyl]-4-(ethyl-1,3,4-oxadiazolyl)-4-(N-phenylpropionamido)piperidinium oxalate | 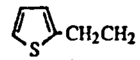 | $CH_3CH_2$ | $CH_3CH_2$ | H | 160-162 | 0.272 |
| 1-[2-(4-ethyl-4,5-dihydro-5-oxo-1H-tetrazol-1-yl)-ethyl]-4-(ethyl-1,3,4-oxadiazolyl)-4-(N-phenyl-propionamido)piperidinium oxalate | 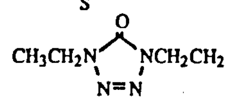 | $CH_3CH_2$ | $CH_3CH_2$ | H | 175-177 | >5.0 |
| 1-[2-(3-thienyl)ethyl]-4-(ethyl-1,3,4-oxadiazolyl)-4-(N-phenylpropionamido)piperidinium oxalate | 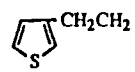 | $CH_3CH_2$ | $CH_3CH_2$ | H | 178-180 | 0.348 |
| 1-[2-(2-pyridinyl)ethyl]-4-(ethyl-1,3,4-oxadiazolyl)-4-(N-phenylpropionamido)piperidinium oxalate | 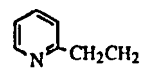 | $CH_3CH_2$ | $CH_3CH_2$ | H | 164-166 | 1.65 |
| 1-(2-ethoxyethyl)-4-(ethyl-1,3,4-oxadiazolyl)-4-(N-phenylpropionamido)piperidinium oxalate | $CH_3CH_2OCH_3CH_2$ | $CH_3CH_2$ | $CH_3CH_2$ | H | 207-210 | >5.0 |
| 1-(2-oxo-2-phenylethyl)-4-(ethyl-1,3,4-oxadiazolyl)-4-(N-phenylpropionamido)piperidinium oxalate | 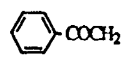 | $CH_3CH_2$ | $CH_3CH_2$ | H | 134-136 | >5.0 |
| 1-[2-(4-trifluoromethyl)phenylethyl]-4-(ethyl-1,3,4-oxadiazolyl)-4-(N-phenylpropionamido)piperidinium oxalate | 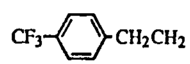 | $CH_3CH_2$ | $CH_3CH_2$ | H | 167-169 | 0.49 |

Table I....continued

| Compound | L | R' | $R^3$ | X | M.P. (°C) | $ED_{50}$mg/kg mouse hot plate |
|---|---|---|---|---|---|---|
| 1-[2-(1-pyrazolyl)ethyl]-4-(ethyl-1,3,4-oxadiazolyl)-4-(N-phenylpropionamido)piperidinium oxalate | 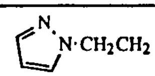 | $CH_3CH_2$ | $CH_3CH_2$ | H | 185-187 | >5.0 |
| 1-(2-phenylethyl)-4-(ethyl-1,3,4-oxadiazolyl)-4-[N-(2-fluorophenyl)propionamido]piperidinium chloride | 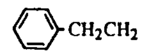 | $CH_3CH_2$ | $CH_3CH_2$ | F | 95-102 | 0.018 |
| 1-[2-(2-thienyl)ethyl]-4-(ethyl-1,3,4-oxadiazolyl)-4-[N-(2-fluorophenyl)propionamido]piperidinium chloride | 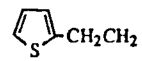 | $CH_3CH_2$ | $CH_3CH_2$ | F | 85-90 | 0.063 |
| 1-[2-(3-thienyl)ethyl]-4-(ethyl-1,3,4-oxadiazolyl)-4-[N-(2-fluorophenyl)propionamido]piperidinium chloride | 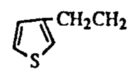 | $CH_3CH_2$ | $CH_3CH_2$ | F | 70-75 | 0.171 |
| 1-[2-(2-pyridinyl)ethyl]-4-(ethyl-1,3,4-oxadiazolyl)-4-[N-(2-fluorophenyl)propionamido)piperidinium chloride | 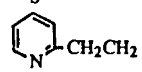 | $CH_3CH_2$ | $CH_3CH_2$ | F | 131-139 | >5.0 |
| 1-(2-phenylethyl)-4-(methyl-1,3,4-oxadiazolyl)-4-(N-phenylpropionamido)piperidinium oxalate | 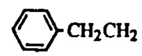 | $CH_3$ | $CH_3CH_2$ | H | 205-207 | 0.027 |
| 1-[2-(2-thienyl)ethyl]-4-(methyl-1,3,4-oxadiazolyl)-4-(N-phenylpropionamido)piperidinium oxalate | 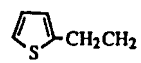 | $CH_3$ | $CH_3CH_2$ | H | 202-203 | 0.10 |
| 1-[2-(3-thienyl)ethyl]-4-(methyl-1,3,4-oxadiazolyl)-4-(N-phenylpropionamido)piperidinium oxalate | 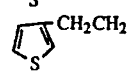 | $CH_3$ | $CH_3CH_2$ | H | 198-200 | 0.016 |
| 1-[2-(4-fluorophenyl)ethyl]-4-(methyl-1,3,4-oxadiazolyl)-4-(N-phenylpropionamido)piperidinium oxalate | 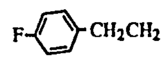 | $CH_3$ | $CH_3CH_2$ | H | 200-202 | 0.242 |
| 1-[2-(4-trifluorophenyl)ethyl]-4-(methyl-1,3,4-oxadiazol-yl)-4-(N-phenylpropionamido)piperidinium oxalate | 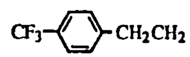 | $CH_3$ | $CH_3CH_2$ | H | 130-132 | 0.309 |
| 1-[2-(2-pyridinyl)ethyl]-4-(methyl-1,3,4-oxadiazolyl)-4-(N-phenylpropionamido)piperidinium oxalate | 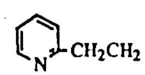 | $CH_3$ | $CH_3CH_2$ | H | 176-177 | >1.0 |
| 1-[2-(2-fluorophenyl)ethyl]-4-(methyl-1,3,4-oxadiazolyl)-4-(N-phenylpropionamido)piperidinium oxalate | 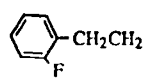 | $CH_3$ | $CH_3CH_2$ | H | 196-197 | 0.0049 |

Table I...continued

| Compound | L | R' | $R^3$ | X | M.P. (°C) | $ED_{50}$mg/kg mouse hot plate |
|---|---|---|---|---|---|---|
| 1-[2-(4-ethyl-4,5-dihydro-5-oxy-1H-tetrazol-1-yl)ethyl]-4-(methyl-1,3,4-oxadiazolyl)-4-(N-phenylpropionamido)piperidinium oxalate | 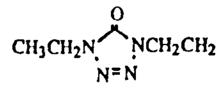 | $CH_3$ | $CH_3CH_2$ | H | 162-165 | 0.772 |
| 1-[2-(1-pyrazolyl)ethyl]-4-(methyl-1,3,4-oxadiazolyl)-4-(N-phenylpropionamido)piperidinium oxalate | 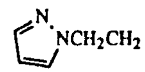 | $CH_3$ | $CH_3CH_2$ | H | 183-184 | 0.063 |
| 1-[2-(N-phthalimido)ethyl]-4-(methyl-1,3,4-oxadiazolyl)-4-(N-phenylpropionamido)piperidinium oxalate | 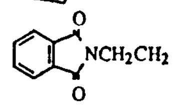 | $CH_3$ | $CH_3CH_2$ | H | 164-167 | >5.0 |
| 1-(2-phenylethyl)-4-(methyl-1,3,4-oxadiazolyl)-4-[N-(2-fluorophenyl)propionamido]piperidinium chloride | 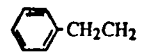 | $CH_3$ | $CH_3CH_2$ | F | 133-137 | 0.016 |
| 1-[2-(2-pyridinyl)ethyl]-4-(propyl-1,3,4-oxadiazolyl)-4-[N-(2-fluorophenyl)propionamido]piperidinium chloride | 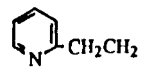 | $CH_3$ | $CH_3CH_2$ | F | 161-163 | 0.525 |
| 1-(2-methylbutyl)-4-(methoxymethyl-1,3,4-oxadiazolyl)-4-(N-phenylpropionamido)piperidinium oxalate | $CH_3CH_2CHCH_3CH_2$ | $CH_3$ | $CH_3CH_2$ | H | 182-183 | 0.484 |
| 1-[2-(2-thienyl)ethyl]-4-(methyl-1,3,4-oxadiazolyl)-4-[N-(2-fluorophenyl)propionamido]piperidinium chloride | 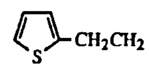 | $CH_3$ | $CH_3CH_2$ | F | 120-125 | 0.017 |
| 1-[2-(1-pyrazolyl)ethyl]-4-(methyl-1,3,4-oxadiazolyl)-4-[N-(2-fluorophenyl)propionamido]piperidinium chloride | 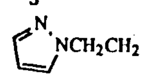 | $CH_3$ | $CH_3CH_2$ | F | 120-123 | >5.0 |
| 1-(2-phenylethyl)-4-(propyl-1,3,4-oxadiazolyl)-4-(N-phenylpropionamido)piperidinium chloride | 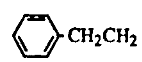 | $CH_3CH_2CH_2$ | $CH_3CH_2$ | H | 110-114 | 0.0017 |
| 1-[2-(2-thienyl)ethyl]-4-(propyl-1,3,4-oxadiazolyl)-4-(N-phenylpropionamido)piperidinium chloride | 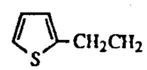 | $CH_3CH_2CH_2$ | $CH_3CH_2$ | H | 105-110 | 0.0014 |
| 1-[2-(4-fluorophenyl)ethyl]-4-(propyl-1,3,4-oxadiazolyl)-4-(N-phenylpropionamido)piperidinium chloride | 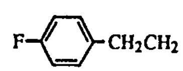 | $CH_3CH_2CH_2$ | $CH_3CH_2$ | H | 111-115 | 0.0082 |

Table I...continued

| Compound | L | R' | $R^3$ | X | M.P. (°C) | $ED_{50}$mg/kg mouse hot plate |
|---|---|---|---|---|---|---|
| 1-[2-(1-pyrazolyl)ethyl]-4-(propyl-1,3,4-oxadiazolyl)-4-(N-phenylpropionamido)piperidinium oxalate | 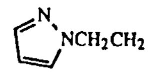 | $CH_3CH_2CH_2$ | $CH_3CH_2$ | H | 112-115 | 0.276 |
| 1-[2-(4-trifluorophenyl)ethyl]-4-(propyl-1,3,4-oxadiazolyl)-4-(N-phenylpropionamido)piperidinium chloride | 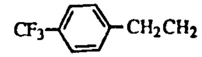 | $CH_3CH_2CH_2$ | $CH_3CH_2$ | H | 107-111 | 0.08 |
| 1-[2-(2-pyridinyl)ethyl]-4-(propyl-1,3,4-oxadiazolyl)-4-(N-phenylpropionamido)piperidinium oxalate | 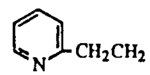 | $CH_3CH_2CH_2$ | $CH_3CH_2$ | H | 150-155 | >1.0 |
| 1-(2-methylbutyl)-4-(propyl-1,3,4-oxadiazolyl)-4-[N-(2-fluorophenyl)propionamido]piperidinium chloride | $CH_3CH_2CHCH_3CH_2$ | $CH_3CH_2CH_2$ | $CH_3CH_2$ | F | 110-113 | 0.195 |
| 1-[2-(4-ethyl-4,5-dihydro-5-oxy-1H-tetrazol-1-yl)-ethyl]-4-(propyl-1,3,4-oxadiazolyl)-4-[N-(2-fluorophenyl)propionamido]piperidinium chloride | 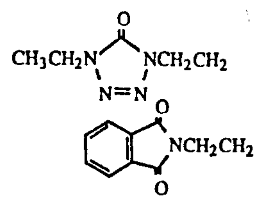 | $CH_3CH_2CH_2$ | $CH_3CH_2$ | F | 119-121 | >1.0 |
| 1-[2-(N-phthalimido)ethyl]-4-(propyl-1,3,4-oxadiazolyl)-4-[N-(2-fluorophenyl)propionamido]piperidinium chloride | | $CH_3CH_2CH_2CH_2$ | $CH_3CH_2$ | H | 145-150 | >1.0 |
| 1-(2-phenylethyl)-4-(butyl-1,3,4-oxadiazolyl)-4-(N-phenylpropionamido)piperidinium chloride | 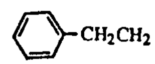 | $CH_3CH_2CH_2CH_2$ | $CH_3CH_2$ | H | 109-110 | 0.0017 |
| 1-[2-(1-pyrazolyl)ethyl]-4-(butyl-1,3,4-oxadiazolyl)-4-(N-phenylpropionamido)piperidinium chloride | 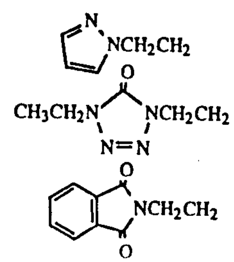 | $CH_3CH_2CH_2CH_2$ | $CH_3CH_2$ | H | 99-103 | >1.0 |
| 1-[2-(4-ethyl-4,5-dihydro-5-oxy-1H-tetrazol-1-yl)-ethyl]-4-(butyl-1,3,4-oxadiazolyl)-4-(N-phenylpropionamido)piperidinium chloride | | $CH_3CH_2CH_2CH_2$ | $CH_3CH_2$ | H | 108-110 | 0.149 |
| 1-[2-(N-phthalimido)ethyl]-4-(butyl-1,3,4-oxadiazolyl)-4-(N-phenylpropionamido)piperidinium chloride | | $CH_3CH_2CH_2CH_2$ | $CH_3CH_2$ | H | 130-135 | >1.0 |

Table I...continued

| Compound | L | R' | $R^3$ | X | M.P. (°C) | $ED_{50}$mg/kg mouse hot plate |
|---|---|---|---|---|---|---|
| 1-[2-(2-thienyl)ethyl]-4-(methoxymethyl-1,3,4-oxadiazolyl)-4-(N-phenylpropionamido)piperidinium chloride | 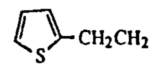 | $CH_3OCH_2$ | $CH_3CH_2$ | H | 121-126 | 0.0007 |
| 1-[2-(2-pyridinyl)ethyl]-4-methoxymethyl-1,3,4-oxadiazolyl)-4-(N-phenylpropionamido)piperidinium oxalate | 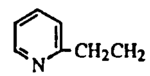 | $CH_3OCH_2$ | $CH_3CH_2$ | H | 157-158 | >1.0 |
| 1-(2-methylpropyl)-4-(methoxymethyl-1,3,4-oxadiazolyl)-4-(N-phenylpropionamido)piperidinium oxalate | $CH_3CHCH_3CH_2$ | $CH_3OCH_2$ | $CH_3CH_2$ | H | 214-215 | >1.0 |
| 1-(phenylethyl)-4-(methoxymethyl-1,3,4-oxadiazolyl)-4-(N-phenylpropionamido)piperidinium chloride | 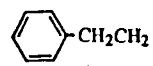 | $CH_3OCH_2$ | $CH_3CH_2$ | H | 115-120 | 0.0023 |
| 1-[2-(4-ethyl-4,5-dihydro-5-oxo-1H-tetrazol-1-yl)ethyl]-4-(methoxymethyl-1,3,4-oxadiazolyl)-4-(N-phenylpropionamido)piperidinium oxalate | 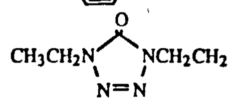 | $CH_3OCH_2$ | $CH_3CH_2$ | H | 153-154 | >5.0 |
| 1-[2-(4-methylthiazol-5-yl)ethyl]-4-(methoxymethyl-1,3,4-oxadiazolyl)-4-(N-phenylpropionamido)-piperidinium oxalate | 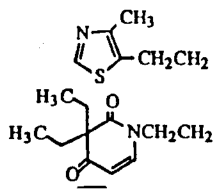 | $CH_3OCH_2$ | $CH_3CH_2$ | H | 227-228 | 0.718 |
| 1-[2-(3,3-diethyl-2,4(1H,3H)-pyridinedion-1-yl)ethyl]-4-(methoxymethyl-1,3,4-oxadiazolyl)-4-(N-phenylpropionamido)piperidinium oxalate | | $CH_3OCH_2$ | $CH_3CH_2$ | H | 160-161 | >1.0 |

Table II

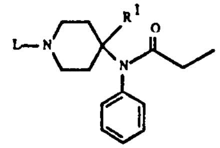

| Compound | L | R¹ | M.P. (°C) | $ED_{50}$mg/kg mouse hot plate |
|---|---|---|---|---|
| 1-(2-phenylethyl)-4-(1-methylimidazol-2-yl)-4-(N-phenylpropionamido)piperidinium oxalate | 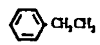 | 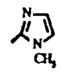 | 213-215 | 0.14 |
| 1-[2-(2-thienyl)ethyl]-4-(1-methylimidazol-2-yl)-4-(N-phenylpropionamido)piperidinium oxalate | 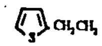 | 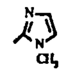 | 208-210 | 0.0096 |
| 1-[2-(1-pyrazolyl)ethyl]-4-(1-methylimidazol-2-yl)-4-(N-phenylpropionamido)piperidinium oxalate | 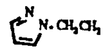 | 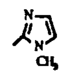 | 195-197 | >5.0 |
| 1-[2-(4-ethyl-4,5-dihydro-5-oxo-1H-tetrazol-1-yl)ethyl]-4-(1-methylimidazol-2-yl)-4-(N-phenylpropionamido)piperidinium oxalate | 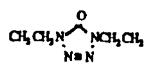 | 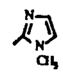 | 144-145 | >5.0 |
| 1-(2-phenylethyl)-4-(1-methyl-1,2,4-triazol-5-yl)-4-(N-phenylpropionamido)piperidinium oxalate | 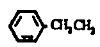 | 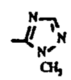 | 206-207 | 0.003 |
| 1-[2-(2-thienyl)ethyl]-4-(1-methyl-1,2,4-triazol-5-yl)-4-(N-phenylpropionamido)piperidinium oxalate | 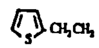 | 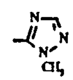 | 216-217 | 0.0023 |
| 1-[2-(4-methylthiazol-5-yl)ethyl]-4-(1-methyl-1,2,4-triazol-5-yl)-4-(N-phenylpropionamido)piperidinium oxalate | 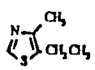 | 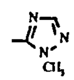 | 218-220 | 0.039 |

Table II...continued

| Compound | L | $R^1$ | M.P. (°C) | $ED_{50}$mg/kg mouse hot plate |
|---|---|---|---|---|
| 1-(2-methylbutyl)-4-(1-methyl-1,2,4-triazol-5-yl)-4-(N-phenylpropionamido)-piperidinium oxalate | $CH_3CH_2CHCH_3CH_2$ | 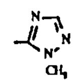 | 132-133 | 0.043 |
| 1-[2-(1-pyrazolyl)ethyl]-4-(1-methyl-1,2,4-triozol-5-yl)-4-(N-phenylpropionamido)-piperidinium oxalate | 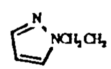 | 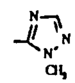 | 186-187 | >1.0 |
| 1-[2-(N-phthalimido)ethyl]-4-(1-methyl-1,2,4-triazol-5-yl)-4-(N-phenylpropionamido)-piperidinium oxalate | 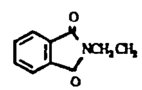 | 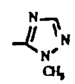 | 168-170 | >1.0 |
| 1-[2-(4-ethyl-4,5-dihydro-5-oxo-1H-tetrazol-1-yl)ethyl]-4-(1-methyl-1,2,4-triazol-5-yl)-4-(N-phenylpropionamido)-piperidinium oxalate | 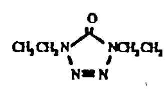 | 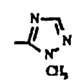 | 211-212 | >1.0 |

Table III

| | Compound | MP °C | Analgesic Activity ($ED_{50}$)mg/kg Mice |
|---|---|---|---|
| 1. | 1-(2-phenylethyl)-4-(4-methylthiazol-2-yl)-4-[N-fluorophenyl)propionamido]piperidinium oxalate | 203.5-204.5 | 0.022 |
| 2. | 1-[2-(4-ethyl-4,5-dihydro-5-oxo-1H-tetrazol-1-yl)ethyl]-4-(4-methyl-thiazol-2-yl)-4-(N-phenylpropionamido)piperidinium oxalate | 154-55 | 0.264 |
| 3. | 1-[2-(4-ethyl-4,5-dihydro-5-oxo-1H-tetrazol-1-yl)ethyl]-4-(4-methylthiazol-2-yl)-4-[N-(2-fluorophenyl)propionamido] piperidinium oxalate | 144-46 | 0.179 |
| 4. | 1-[2-(1H-pyrazol-1-yl)ethyl]-4-(4-methyl-thiazol-2-yl)-4-(N-phenyl-propionamido) piperidinium oxalate | 185-87 | 0.064 |
| 5. | 1-[2-1H-pyrazol-1-yl)ethyl]-4-(4-methyl-thiazol-2-yl)-4-[N-(2-fluoro-phenyl) propionamide] piperidinium oxalate | 185-87 | 0.047 |
| 6. | 1-[2-(2-thienyl)ethyl]-4-(4-methyl-thiazol-2-yl)-4-[N-(2-fluorophenyl) propionamido] piperidinium oxalate | 198-200 | 0.012 |
| 7. | 1-[2-(3-thienyl)ethyl]-4-(4-methyl-thiazol-2-yl)-4-[N-(2-fluorophenyl) propionamido] piperidinium oxalate | 202-204 | 0.0054 |
| 8. | 1-(2-phenylethyl)-4-(2-thiazolyl)-4-(N-phenyl propionamido) piperidinium oxalate | 193-94 | 0.0047 |
| 9. | 1-(2-phenylethyl)-4-(2-thiazolyl)-4-[N-(2-fluorophenyl)propionamido] piperidinium oxalate | 202-203 | 0.0034 |
| 10. | 1-[2-(4-ethyl-4,5-dihydro-5-oxo-1H-tetrazol-1-yl)ethyl]-4-(2-thiazolyl)-4-[N-phenylpropionamido) piperidinium oxalate | 169-70 | 0.212 |

Table III continued

| | Compound | MP °C | Analgesic Activity ($ED_{50}$)mg/kg Mice |
|---|---|---|---|
| 11. | 1-[2-(4-ethyl-4,5-dihydro-5-oxo-1H-tetrazol-1-yl)ethyl]-4-(2-thiazolyl)-4-[N-(2-fluorophenyl)propionamido] piperidinium oxalate | 177-78 | 0.057 |
| 12. | 1-(2-phenylethyl)-4-(4,5-dimethyl-thiazol-2-yl)-4-(N-phenylpropionamido) piperidinium oxalate | 191-192 | 5.0 |
| 13. | 1-[2-(3-thienyl)ethyl]-4-(4,5-dimethyl-thiazol-2-yl)-4-(N-phenylpropionamido) piperidinium oxalate | 201-202 | 0.525 |
| 14. | 1-[2-(4-ethyl-4,5-dihydro-5-oxo-1H-tetrazol-1-yl)ethyl]-4-(4,5-dimethyl-thiazol-2-yl)-4-(N-phenylpropionamido) piperidinium oxalate | 194-95 | N/A |
| 15. | 1-(2-phenylethyl)-4-(4-methylthiazol-2-yl)-4-(N-phenylpropionamido) piperidinium oxalate | 204 | 0.072 |
| 16. | 1-[2-(2-thienyl)ethyl]-4-(4-methylthiazol-2-yl)-4-(N-phenylpropionamido) piperidinium oxalate | 181 | 0.01 |
| 17. | 1-[2-(3-thienyl)ethyl]-4-(4-methylthiazol-2-yl)-4-(N-phenylpropionamido) piperidinium oxalate | 202-204 | 0.0059 |
| 18. | 1-[2-(4-methylthiazol-5-yl)ethyl]-4-(4-methylthiazol-2-yl)-4-[N-(2-fluorophenyl) propionamido] piperidinium oxalate | 225 | 0.017 |

## Claims

**1.** A compound of the formula

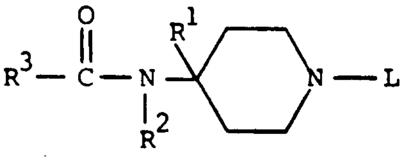

(I)

and/or a pharmaceutically acceptable acid addition salt thereof, in which formula:
$R^1$ is a thiazol-2-yl, 4-methyl-thiazol-2-yl, or 4,5-dimethylthiazol-2-yl group; a substituted 1,3,4-oxadiazolyl group wherein the substituent is a methyl, ethyl, n-propyl, n-butyl or methoxy-methyl group; a 1-methyl-imidazol-2-yl group; or 1-methyl-1 H-1,2,4-triazol-5-yl group;
$R^2$ is phenyl or 2-fluorophenyl;

$R^3$ is a methyl or ethyl group; and
L is a 2-phenylethyl, 1-phenyl-2-propyl, 2-phenyl-1-propyl, (4-ethyl-4,5-dihydro-5-oxo-1H-tetrazol-1-yl) ethyl, thiazol ethyl, benzyl, 2-(2-thienyl)ethyl, 2-(3-thienyl)ethyl, 2-(1H-pyrazol-1-yl)ethyl, 2-(2-pyridinyl)-ethyl, 2-methylbutyl, ethyl-1,3,4-oxadiazolyl, 2-ethoxyethyl, 2-oxo-2-phenylethyl, 2-(4-trifluoromethyl)-phenylethyl, 2-(4-fluorophenyl)ethyl, 2-(2-fluorophenyl)ethyl, 2-N-phthalimido)ethyl, 2-methylpropyl, 2-(4-methylthiazol-5-yl)ethyl, or (3,3-diethyl-2,4-(1H,3H) pyridinedion-1-yl)ethyl group.

**2.** 1-(2-Phenylethyl)-4-(ethyl-1,3,4-oxadiazolyl)-4-(N-phenyl-propionamido)piperidine or a pharmaceutically acceptable acid addition salt thereof.

**3.** 1-[2-(2-Thienyl)ethyl]-4-(ethyl-1,3,4-oxadiazolyl)-4-(N-phenylpropionamido)piperidine or a pharmaceutically acceptable acid addition salt thereof.

**4.** 1-Benzyl-4-(4-methyl-thiazol-2-yl)-4-(N-phenylpropionamido)piperidine or a pharmaceutically acceptable acid addition salt thereof.

**5.** 1-[2-(1H-Pyrazol-1-yl)ethyl]-4-(4-methylthiazol-2-yl)-4-(N-phenylpropionamido)piperidine or a pharmaceutically acceptable acid addition salt thereof.

**6.** A compound or salt according to claim 1, selected from: 1-[2-(3-thienyl)ethyl]-4-(ethyl-1,3,4-oxadiazolyl)-4-(N-phenylpropionamido) piperidine; 1-(2-phenylethyl)-4-(methyl-1,3,4-oxadiazolyl)-4-(N-phenylpropionamido)piperidine; 1-[2-(4-ethyl-4,5-dihydro-5-oxo-1H-tetrazol-1-yl)ethyl]-4-(methyl-1,3,4-oxadiazolyl)-4-(N-phenylpropionamido)piperidine; 1-(2-phenylethyl)-4-(propyl-1,3,4-oxadiazolyl)-4-(N-phenylpropionamido)piperidine; 1-[2-(2-thienyl)ethyl]-4-(propyl-1,3,4-oxadiazolyl)-4-(N-phenylpropionamido)piperidine; 1-(2-phenylethyl)-4-(butyl-1,3,4-oxadiazolyl)-4-(N-phenylpropionamido)piperidine; 1-[2-(2-thienyl)ethyl]-4-(methoxymethyl-1,3,4-oxadiazolyl)-4-(N-phenyl-propionamido) piperidine; 1-(2-phenylethyl)-4-(methoxymethyl-1,3,4-oxadiazolyl)-4-(N-phenylpropionamido)piperidine; 1-[2-(4-methylthiazol-5-yl)ethyl]-4-(methoxymethyl-1,3,4-oxadiazolyl)-4-(N-phenylpropionamido)piperidine; 1-(2-phenylethyl)-4-(1-methyl-1,2,4-triazol-5-yl)-4-(N-phenylpropionamido)piperidine; 1-[2-(4-methylthiazol-5-yl)ethyl]-4-(methyl-1,2,4-triazol-5-yl)-4-(N-phenylpropionamido)piperidine; and pharmaceutically acceptable acid addition salts thereof.

**7.** A compound according to any preceding claim for use as an analgesic or anaesthetic.

**8.** An analgesic or anaesthetic composition comprising a compound according to any of claims 1 to 6 and a pharmaceutically acceptable carrier.

**9.** A method of preparing a compound of formula (I) according to claim 1, wherein
(a) a compound of formula

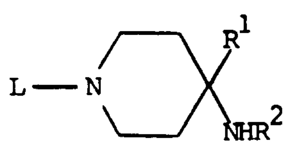

(II)

is reacted with an acylating agent containing an $R^3$ group to form said compound of formula (I); or
(b) a compound of formula

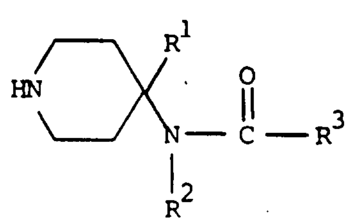

is reacted with a compound containing a L group to substitute the L group for the hydrogen bonded to the nitrogen atom of the piperidine moiety and thereby form the compound of formula (I).

**10.** A method according to claim 9, in which the compound of formula (II) is prepard by reacting a piperidone of formula:

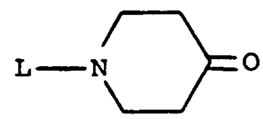

with an amine of formula $R^2NH_2$ to form a Schiff base of formula:

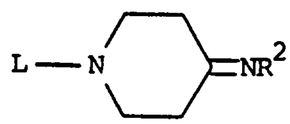

and reacting the Schiff base with a compound of formula $R^1Li$ to form the compound of formula (II).

**11.** A method according to claim 9, in which the compound of formula (II) is formed by reacting an amine of formula $R^2NH_2$ and an inorganic cyanide salt with a piperidone of formula:

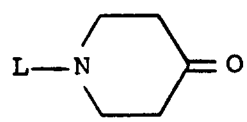

to form a cyano intermediate of formula:

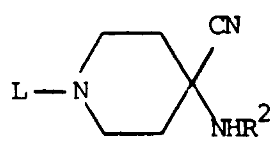

and then substituting an $R^1$ group for the cyano group to form said compound of formula (II).

**12.** A method according to claim 11, in which the substitution is performed by reacting said cyano intermediate with a compound of formula $R^1Li$.

**13.** A method according to claim 9, in which the compound of formula

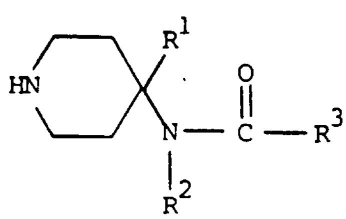

is prepared by catalytically hydrogenating a compound of the formula:

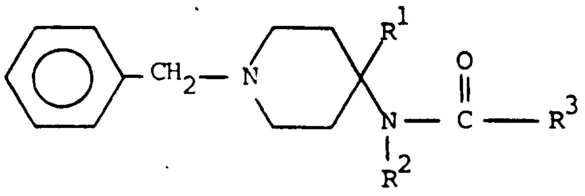

(III)

or by reacting said compound of formula (III) with alphychloroethyl chloroformate and then methandysing the resulting mixture, wherein said compound of formula (III) is prepared by the method of any of claims 9 to 11.

**14.** A method according to claim 9, in which in the compound of formula:

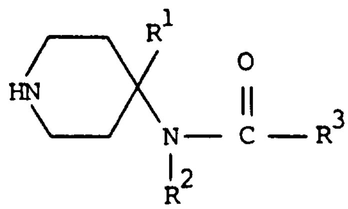

$R^1$ is a 1,3,4-oxadiazolyl group substituted with an $R^3$ group, and is prepared by reacting a compound of formula:

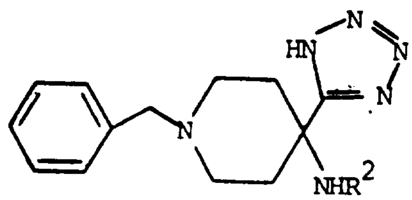

(IV)

with an acylating agent containing an $R^3$ moiety in its acyl group under conditions to form a compound of formula:

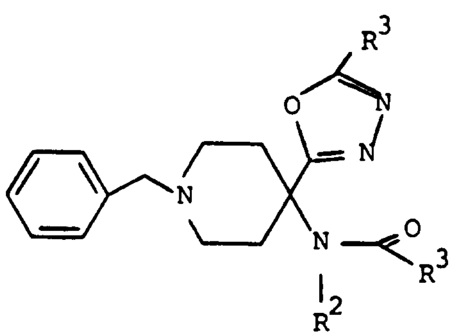

(V)

and then catalytically hydrogenating said compound of formula (V).

**15.** A method according to claim 14, in which the acylating agent is an acid anhydride of formula $(R^3CO)_2O$ and its reaction takes place in the temperature range of 110 to 160 °C.

**16.** A method according to claim 9, in which in the compound of formula:

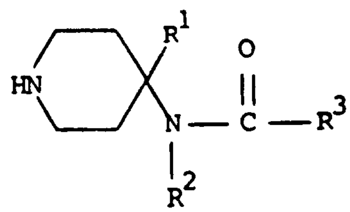

the $R^1$ group is a 1,3,4-oxadiazolyl group substituted with an $R^4$ group, and the compound is prepared by reacting a compound of formula:

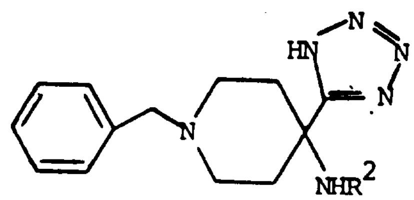

(IV)

with a first acylating agent including an $R^3$ group under conditions to form a compound of formula:

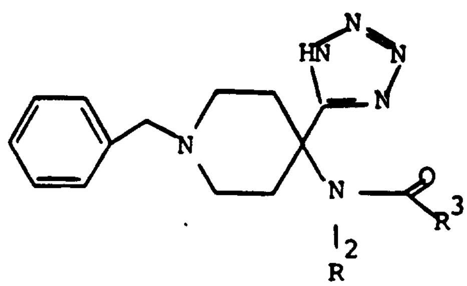

(VI)

and reacting the compound of formula (VI) with a second acylating agent including an $R^4$ group under conditions to form a compound of formula:

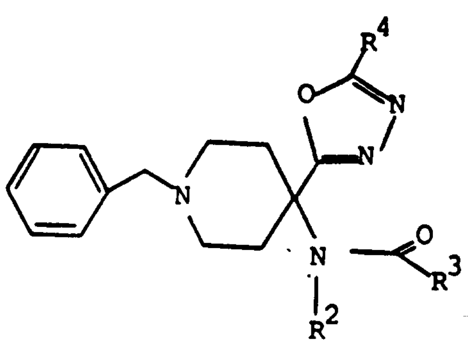

(VII)

wherein $R^4$ is methyl, ethyl, n-propyl, n-butyl or methoxymethyl and different from $R^3$.

**17.** A method according to claim 16, in which the first acylating agent is an acid anhydride of formula $(R^3CO)_2O$ and its reaction takes place in the temperature range of 70 to 75°C, and the second acylating agent is an acide anhydride of formula $(R^4CO)_2O$ and its reaction takes place in the temperature range of 110 to 160°C.

**18.** A method according to claim 14 or 16, wherein the compound of formula (IV) is prepared by converting the cyano group in a compound of formula:

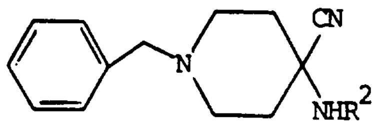

to a tetrazol group.

**19.** A method according to claim 18, in which the cyano (or nitrile) group is converted to the tetrazolyl group by reaction with an azide salt in a non-aqueous reaction medium.

**20.** A method according to claim 19, in which the azide salt is sodium azide.

**21.** A method according to claim 19 or 20, in which the reaction medium is provided by tetrahydrofuran.

**22.** A method according to any of claims 18 to 21, in which the compound of formula:

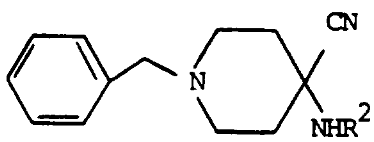

is formed by reacting N-benzyl piperidone with a cyanide salt and with an amine of formula $R^2NH_2$.

**23.** A method of preparing an analgesic or anaesthetic composition comprising bringing a compound according to any of claims 1 to 6 into admixture with a pharmaceutically acceptable carrier.

**Patentansprüche**

**1.** Verbindung der Formel

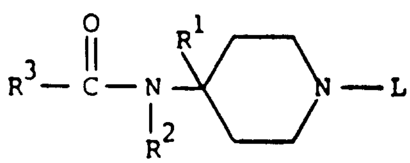

(I)

und/oder ein pharmazeutisch akzeptables Säureadditionssalz davon, wobei in der Formel:
$R^1$ eine Thiazol-2-yl-, 4-Methyl-thiazol-2-yl- oder 4,5-Dimethylthiazol-2-ylgruppe; eine substituierte 1,3,4-Oxadiazolylgruppe, in der der Substituent eine Methyl-, Ethyl-, n-Propyl, n-Butyl oder Methoxymethylgruppe ist; eine 1-Methylimidazol-2-ylgruppe; oder 1-Methyl-1H-1,2,4-triazol-5-ylgruppe ist;
$R^2$ Phenyl oder 2-Fluorphenyl ist;
$R^3$ eine Methyl- oder Ethylgruppe ist; und
L eine 2-phenylethyl-, 1-Phenyl-2-propyl-, 2-Phenyl-1-propyl-, (4-Ethyl-4,5-dihydro-5-oxo-1H-tetrazol-1-yl)ethyl-, Thiazolylethyl-, Benzyl-, 2-(2-Thienyl)ethyl-, 2-(3-Thienyl)-ethyl-,2-(1H-Pyrazol-1-yl)ethyl-, 2-(2-Pyridinyl)ethyl-, 2-Methylbutyl-, Ethyl-1,3,4-oxadiazolyl-, 2-Ethoxyethyl-, 2-Oxo-2-phenylethyl-, 2-(4-Trifluormethyl)phenylethyl-, 2-(4-Fluorphenyl)ethyl-, 2-(2-Fluorphenyl)ethyl-, 2-N-Phthalimido-ethyl-, 2-Methylpropyl-, 2-(4-Methylthiazol-5-yl)ethyl-, oder
(3,3-Diethyl-2,4-(1H,3H)pyridindion-1-yl)ethylgruppe ist.

**2.** 1-(2-Phenylethyl)-4-(ethyl-1,3,4-oxadiazolyl)-4-(N-phenylpropionamido)piperidin oder ein pharmazeutisch akzeptables Säureadditionssalz davon.

**3.** 1-[2-(2-Thienyl)ethyl]-4-(ethyl-1,3,4-oxadiazolyl)-4-(N-phenylpropionamido)piperidin oder ein pharmazeutisch akzeptables Säureadditionssalz davon.

**4.** 1-Benzyl-4-(4-methyl-thiazol-2-yl)-4-(N-phenylpropionamido)piperidin oder ein pharmazeutisch akzeptables Säureadditionssalz davon.

**5.** 1-[2-(1H-Pyrazol-1-yl)ethyl]-4-(4-methylthiazol-2-yl)-4-(N-phenylpropionamido)piperidin oder ein pharmazeutisch akzeptables Säureadditionssalz davon.

**6.** Eine Verbindung oder ein Salz nach Anspruch 1, ausgewählt aus: 1-[2-(3-Thienyl)ethyl]-4-(ethyl-1,3,4-oxadiazolyl)-4-(N-phenylpropionamido)piperidine; 1-(2-Phenylethyl)-4-(methyl-1,3,4-oxadiazolyl)-4-(N-phenylpropionamido)piperidin; 1-[2-(4-ethyl-4,5-dihydro-5-oxo-1H-tetrazol-1-yl)ethyl]-4-(methyl-1,3,4-oxadiazolyl)-4-(N-phenylpropionamido)piperidin; 1-(2-Phenylethyl)-4-(propyl-1,3,4-oxadiazolyl)-4-(N-phenyl-propionamido)piperidine; 1-[2-(2-Thienyl)ethyl]-4-(propyl-1,3,4-oxadiazolyl)-4-(N-phenylpropionamido)piperidin; 1-(2-Phenylethyl)-4-(butyl-1,3,4-oxadiazolyl)-4-(N-phenylpropionamido)piperidin; 1-[2-(2-Thienyl)ethyl]-4-(methoxymethyl-1,3,4-oxadiazolyl)-4-(N-phenylpropionamido)piperidin; 1-(2-Phenylethyl)-4-(methoxymethyl-1,3,4-oxadiazolyl)-4-(N-phenylpropionamido)piperidin; 1-[2-(4-Methylthiazol-5-yl)-ethyl]-4-(methoxymethyl-1,3,4-oxadiazolyl)-4-(N-phenylpropionamdo)piperidin; 1-(2-Phenylethyl)-4-(1-methyl-1,2,4-triazol-5-yl)-4-(N-phenylpropionamido)piperidin; 1-[2-(4-Methylthiazol-5-yl)ethyl]-4-(methyl-1,2,4-triazol-5-yl)-4-(N-phenylpropionamido)piperidin; und pharmazeutisch akzeptable Säureadditionssalze davon.

**7.** Eine Verbindung nach einem vorausgehenden Ansprüche zur Verwendung als Analgetikum oder Anästhetikum.

**8.** Eine analgetische oder anästhetische Zusammensetzung, die eine Verbindung nach einem der Ansprüche 1 bis 6 und einen pharmazeutisch akzeptablen Träger enthält.

**9.** Verfahren zur Herstellung einer Verbindung der Formel (I) nach Anspruch 1, bei dem
(a) eine Verbindung der Formel

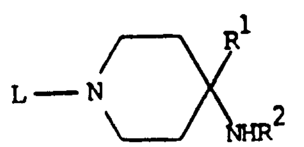

(II)

mit einem Acylierungsmittel, das eine $R^3$-Gruppe enthält, unter Bildung der Verbindung der Formel (I) umgesetzt wird; oder
(b) eine Verbindung der Formel

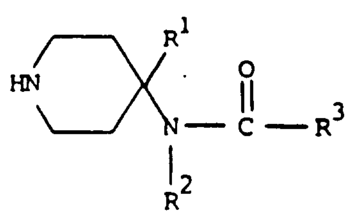

mit einer Verbindung, die eine L-Gruppe enthält, umgesetzt wird, um den an das Stickstoffatom des Piperidinrests gebundenen Wasserstoff durch die L-Gruppe zu ersetzen und dadurch die Verbindung der Formel (I) zu bilden.

**10.** Verfahren nach Anspruch 9, bei dem die Verbindung der Formel (II) durch Umsetzung eines Piperidons der Formel:

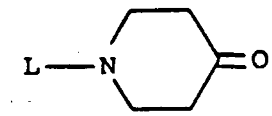

mit einem Amin der Formel $R^2NH_2$ unter Bildung einer Schiffschen Base der Formel:

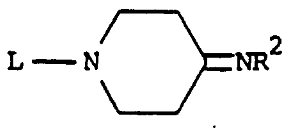

hergestellt wird und die Schiffsche Base mit einer Verbindung der Formel $R^1Li$ unter Bildung der Formel (II) umgesetzt wird.

11. Verfahren nach Anspruch 9, bei dem die Verbindung der Formel (II) durch Umsetzung eines Amins der Formel $R^2NH_2$ und eines anorganischen Cyanidsalzes mit einem Piperidon der Formel:

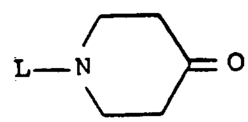

unter Bildung eines Cyanozwischenproduktes der Formel:

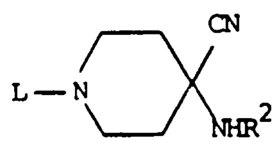

gebildet wird und dann die Cyanogruppe durch eine $R^1$-Gruppe unter Bildung der Verbindung der Formel (II) ersetzt wird.

12. Verfahren nach Anspruch 11, bei dem die Substitution durch Umsetzung des Cyanozwischenproduktes mit einer Verbindung der Formel $R^1Li$ durchgeführt wird.

13. Verfahren nach Anspruch 9, bei dem die Verbindung der Formel:

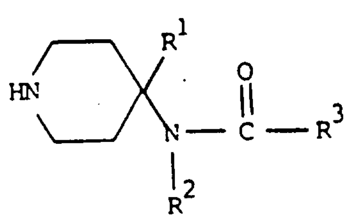

durch katalytisches Hydrieren einer Verbindung der Formel:

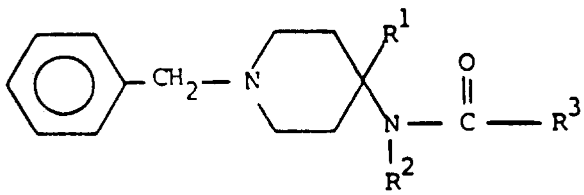 (III)

hergestellt wird oder durch Umsetzung der Verbindung der Formel (III) mit alpha-Chlorethylchlorformiat und Methanolyse der entstandenen Mischung, wobei die Verbindung der Formel (III) durch das Verfahren einer der Ansprüche 9 bis 11 hergestellt wird.

**14.** Verfahren nach Anspruch 9, bei dem in der Verbindung der Formel:

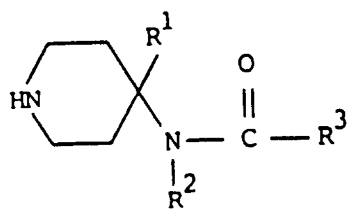

$R^1$ eine mit einer $R^3$-Gruppe substituierte 1,3,4-Oxadiazolylgruppe ist und die Verbindung hergestellt wird durch Umsetzung einer Verbindung der Formel:

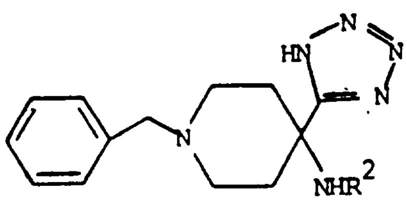

(IV)

mit einem Acylierungsmittel, das einen $R^3$-Rest in seiner Acylgruppe enthält, unter Bedingungen, daß eine Verbindung der Formel:

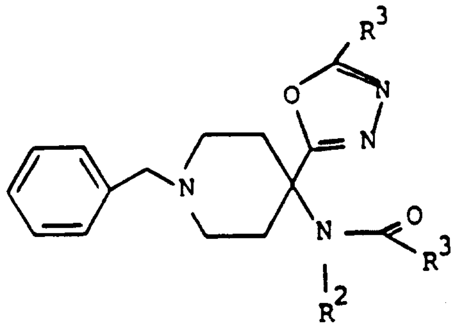

(V)

gebildet wird, und dann durch katalytische Hydrierung der Verbindung der Formel (V).

**15.** Verfahren nach Anspruch 14, bei dem das Acylierungsmittel ein Säureanhydrid der Formel $(R^3CO)_2O$ ist und seine Umsetzung in dem Temperaturbereich von 110 bis 160 °C stattfindet.

**16.** Verfahren nach Anspruch 9, bei dem in der Verbindung der Formel:

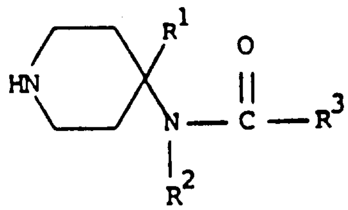

die $R^1$-Gruppe eine mit einer $R^4$-Gruppe substituierte 1,3,4-Oxadiazolylgruppe ist und die Verbindung hergestellt wird durch Umsetzung einer Verbindung der Formel:

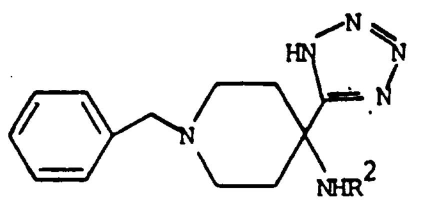

(IV)

mit einem ersten Acylierungsmittel, das eine $R^3$-Gruppe enthält, unter Bedingungen, daß eine Verbindung der Formel:

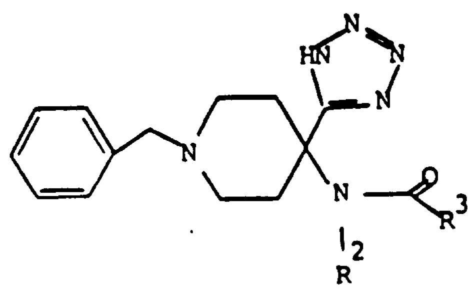

(VI)

gebildet wird, und Umsetzung der Verbindung der Formel (VI) mit einem zweiten Acylierungsmittel, das eine $R^4$-Gruppe enthält, unter Bedingungen, daß eine Verbindung der Formel:

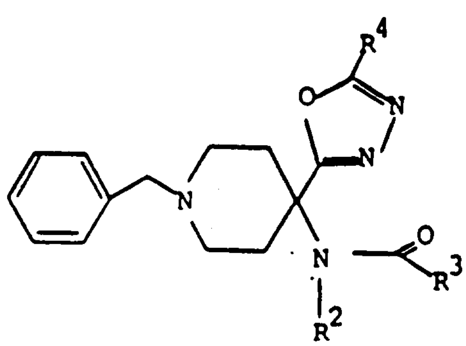

(VII)

gebildet wird, in der $R^4$ Methyl, Ethyl, n-Propyl, n-Butyl oder Methoxymethyl ist und sich von $R^3$ unterscheidet.

**17.** Verfahren nach Anspruch 16, bei dem das erste Acylierungsmittel ein Säureanhydrid der Formel $(R^3CO)_2O$ ist und seine Umsetzung in dem Temperaturbereich von 70 bis 75 °C stattfindet und das

zweite Acylierungsmittel ein Säureanhydrid der Formel $(R^4CO)_2O$ ist und seine Umsetzung in dem Temperaturbereich von 110 bis 160 °C stattfindet.

**18.** Verfahren nach Anspruch 14 oder 16, bei dem die Verbindung der Formel (IV) hergestellt wird durch Umwandlung der Cyanogruppe in einer Verbindung der Formel:

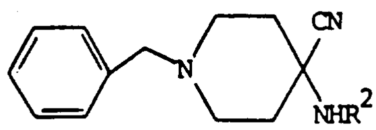

in eine Tetrazolylgruppe.

**19.** Verfahren nach Anspruch 18, bei dem die Cyano-(oder Nitril-) gruppe in eine Tetrazolylgruppe durch Umsetzung mit einem Azidsalz in nichtwäßrigem Reaktionsmedium umgewandelt wird.

**20.** Verfahren nach Anspruch 19, bei dem das Azidsalz Natriumazid ist.

**21.** Verfahren nach Anspruch 19 oder 20, bei dem das Reaktionsmedium Tetrahydrofuran ist.

**22.** Verfahren nach einem der Ansprüche 18 bis 21, bei dem die Verbindung der Formel:

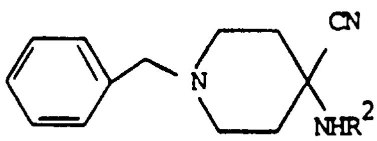

durch Umsetzung von N-Benzylpiperidon mit einem Cyanidsalz und einem Amin der Formel $R^2NH_2$ gebildet wird.

**23.** Verfahren zur Herstellung einer analgetischen oder anästhetischen Zusammensetzung, bei dem eine Verbindung nach einem der Ansprüche 1 bis 6 mit einem pharmazeutisch akzeptablen Träger in Mischung gebracht wird.

**Revendications**

**1.** Composé de formule

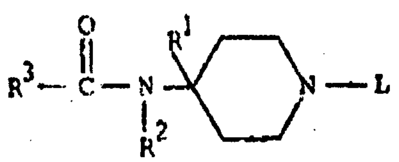

(I)

et/ou un de ses sels d'addition d'acides pharmaceutiquement acceptables, formule dans laquelle :

$R^1$ représente un groupe thiazole-2-yle, 4-méthylthiazole-2-yle ou 4,5-diméthylthiazole-2-yle ; un groupe 1,3,4-oxadiazolyle substitué dans lequel le substituant est un groupe méthyle, éthyle, n-propyle, n-butyle ou méthoxyméthyle ; un groupe 1-méthylimidazole-2-yle ; ou un groupe 1-méthyl-1H-1,2,4-triazole-5-yle ;

$R^2$ représente un groupe phényle ou 2-fluorophényle ;

$R^3$ représente un groupe méthyle ou éthyle ; et

L représente un groupe 2-phényléthyle, 1-phényl-2-propyle, 2-phényl-1-propyle, (4-éthyl-4,5-dihydro-5-oxo-1H-tétrazole-1-yl)éthyle, thiazole-éthyle, benzyle, 2-(2-thiényl)éthyle, 2-(3-thiényl)éthyle, 2-(1H-pyrazole-1-yl)éthyle, 2-(2-pyridinyl)éthyle, 2-méthylbutyle, éthyl-1,3,4-oxadiazolyle, 2-éthoxyéthyle,

2-oxo-2-phényléthyle, 2-(4-trifluorométhyl)phényléthyle, 2-(4-fluorophényl)éthyle, 2-(2-fluorophényl)-éthyle, 2-(N-phtalimido)éthyle, 2-méthylpropyle, 2-(4-méthylthiazole-5-yl)éthyle ou (3,3-diéthyl-2,4-(1H,3H)pyridinedione-1-yl)éthyle.

**2.** 1-(2-phényléthyl)-4-(éthyl-1,3,4-oxadiazolyl)-4-(N-phénylpropionamido)pipéridine ou un de ses sels d'addition d'acides pharmaceutiquement acceptables.

**3.** 1-[2-(2-thiényl)éthyl]-4-(éthyl-1,3,4-oxadiazolyl)-4-(N-phénylpropionamido)pipéridine ou un de ses sels d'addition d'acides pharmaceutiquement acceptables.

**4.** 1-benzyl-4-(4-méthylthiazole-2-yl)-4-(N-phénylpropionamido)pipéridine ou un de ses sels d'addition d'acides pharmaceutiquement acceptables.

**5.** 1-[2-(1H-pyrazole-1-yl)éthyl]-4-(4-méthylthiazole-2-yl)-4-(N-phénylpropionamido)pipéridino ou un de ses sels d'addition d'acides pharmaceutiquement acceptables.

**6.** Composé ou sel suivant la revendication 1, choisi entre :
la 1-[2-(3-thiényl)éthyl]-4-(éthyl-1,3,4-oxadiazolyl)-4-(N-phénylpropionamido)pipéridine ; la 1-(2-phényléthyl)-4-(méthyl-1,3,4-oxadiazolyl)-4-(N-phénylpropionamido)pipéridine ; la 1-[2-(4-éthyl-4,5-dihydro-5-oxo-1H-tétrazole-1-yl)éthyl]-4-(méthyl-1,3,4-oxadiazolyl)-4-(N-phénylpropionamido)pipéridine ; la 1-(2-phényléthyl)-4-(propyl-1,3,4-oxadiazolyl)-4-(N-phénylpropionamido)-pipéridine ; la 1-[2-(2-thiényl)éthyl]-4-(propyl-1,3, 4-oxadiazolyl)-4-(N-phénylpropionamido)pipéridine ; la 1-(2-phényléthyl)-4-(butyl-1,3,4-oxadiazolyl)-4-(N-phénylpropionamido)pipéridine ; la 1-[2-(2-thiényl)éthyl]-4-(méthoxyméthyl-1,3,4-oxadiazolyl)-4-(N-phénylpropionamido)-pipéridine ; la 1-(2-phényléthyl)-4-(méthoxyméthyl-1,3,4-oxadiazolyl)-4-(N-phénylpropionamido)pipéridine ; la 1-[2-(4-méthylthiazole-5-yl)éthyl]-4-(méthoxyméthyl-1,3,4-oxadiazolyl)-4-(N-phénylpropionamido)pipéridine ; la 1-(2-phényléthyl)-4-(1-méthyl-1,2,4-triazole-5-yl)-4-(N-phénylpropionamido)pipéridine ; la 1-[2-(4-méthylthiazole-5-yl)éthyl]-4-(méthyl-1,2,4-triazole-5-yl)-4-(N-phénylpropionamido)pipéridine ; et leurs sels d'addition d'acides pharmaceutiquement acceptables.

**7.** Composé suivant l'une quelconque des revendications précédentes, destiné à être utilisé comme analgésique ou anesthésique.

**8.** Composition analgésique ou anesthésique comprenant un composé suivant l'une quelconque des revendications 1 à 6 et un support pharmaceutiquement acceptable.

**9.** Procédé de préparation d'un composé de formule (I) suivant la revendication 1, dans lequel
(a) un composé de formule

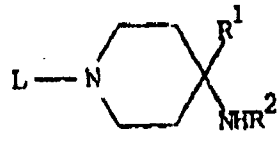

(II)

est amené à réagir avec un agent d'acylation contenent un groupe $R^3$ pour former ledit composé de formule (I) ; ou
(b) un composé de formule

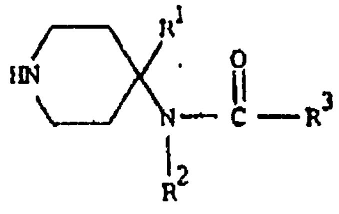

est amené à réagir avec un composé content un groupe L pour remplacer l'atome d'hydrogène lié à l'atome d'azote du groupement pipéridine par le groupe L et former ainsi le composé de formule (I).

**10.** Procédé suivant la revendication 9, dans lequel le composé de formule (II) est préparé par réaction d'une pipéridone de formule :

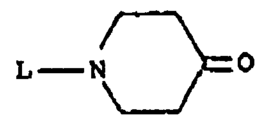

avec une amine de formule $R^2NH_2$ pour former une base de Schiff de formule :

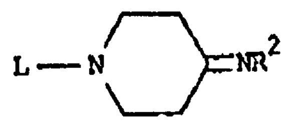

et réaction de la base de Schiff avec un composé de formule $R^1Li$ pour former le composé de formule (II).

**11.** Procédé suivant la revendication 9, dans lequel le composé de formule (II) est formé par réaction d'une amine de formule $R^2NH_2$ et d'un cyanure inorganique avec une pipéridone de formule :

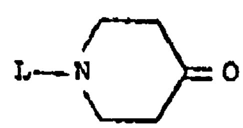

pour former un intermédiaire à fonction cyano, de formule :

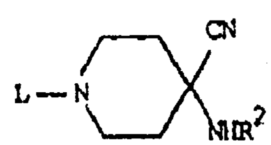

puis par substitution du groupe cyano par un groupe $R^1$ pour former le composé de formule (II).

**12.** Procédé suivant la revendication 11, dans lequel la substitution est effectuéc par réaction de l'intermédiaire à fonction cyano avec un composé de formule $R^1Li$.

**13.** Procédé suivant la revendication 9, dans lequel le composé de formule

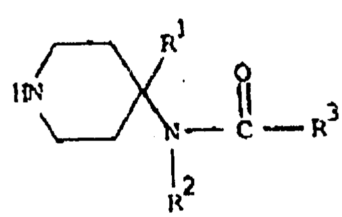

est préparé par hydrogénation catalytique d'un composé de formule :

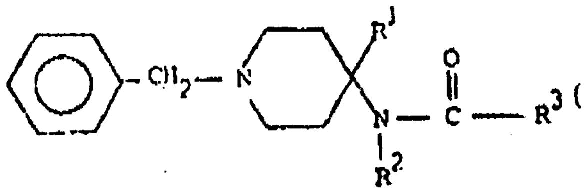

(III)

ou par réaction dudit composé de formule (III) avec le chloroformiate d'alpha-chloréthyle, puis par méthanolysation du mélange résultant, ledit composé de formule (III) étant préparé par le procédé suivant l'une quelconque des revendications 9 à 11.

**14.** Procédé suivant la revendication 9, dans lequel, dans le composé de formule :

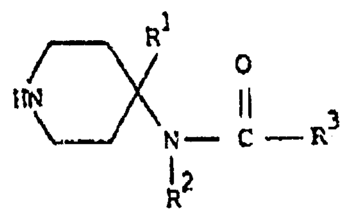

$R^1$ représente un groupe 1,3,4-oxadiazolyle substitué avec un groupe $R^3$, et le composé est préparé par réaction d'un composé de formule :

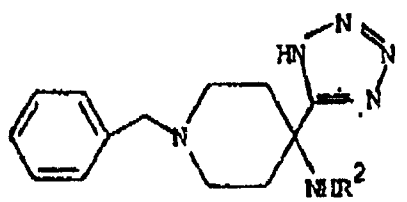

(IV)

avec un agent d'acylation contenant un groupement $R^3$ dans son groupe acyle dans des conditions permettant le formation d'un composé de formule :

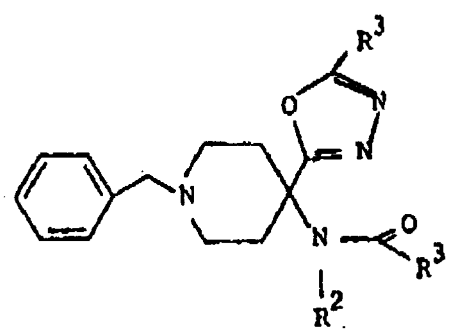

(V)

puis par hydrogénation catalytique dudit composé de formule (V).

**15.** Procédé suivant la revendication 14, dans lequel l'agent d'acylation est un anhydride d'acide de formule $(R^3CO)_2O$ et sa réaction s'effectue dans la plage de températures de 110 à 160 °C.

**16.** Procédé suivant la revendication 9, dans lequel, dans le composé de formule :

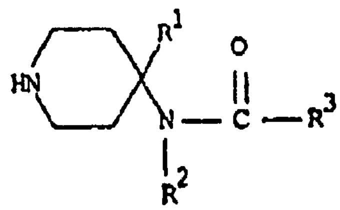

le groupe $R^1$ est un groupe 1,3,4-oxadiazolyle substitué avec un groupe $R^4$, et le composé est préparé par réaction d'un composé de formule :

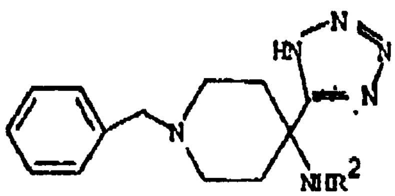

(IV)

avec un premier agent d'acylation comprenant un groupe $R^3$ dans des conditions permettant la formation d'un composé de formule :

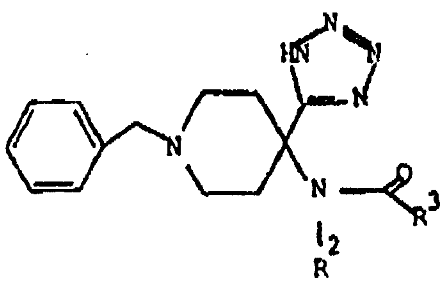

(VI)

et par réaction d'un composé de formule (VI) avec un second agent d'acylation comprenant un groupe $R^4$ dans des conditions permettant la formation d'un composé de formule :

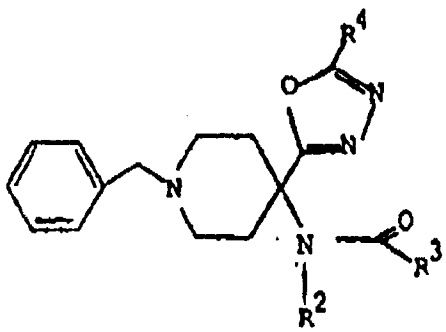

(VII)

dans laquelle $r^4$ représente un groupe méthyle, éthyle, n-propyle, n-butyle ou méthoxyméthyle et est différent de $R^3$.

**17.** Procédé suivant la revendication 16, dans lequel le premier agent d'acylation est un anhydride d'acide de formule $(R^3CO)_2O$ et sa réaction s'effectue dans la plage de températures de 70 à 75 °C, et le second agent d'acylation est un anhydride d'acide de formule $(R^4CO)_2O$ et sa réaction s'effectue dans

le plage de températures de 110 à 160 °C.

**18.** Procédé suivant la revendication 14 ou 16, dans lequel le composé de formule (IV) est préparé par transformation du groupe cyano dans un composé de formule :

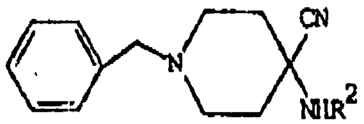

en un groupe tétrazole.

**19.** Procédé suivant la revendication 18, dans lequel le groupe cyano (ou nitrile) est transformé en le groupe tétrazolyle par réaction avec un azoture dans un milieu réactionnel non aqueux.

**20.** Procédé suivant la revendication 19, dans lequel l'azoture est l'azoture de sodium.

**21.** Procédé suivant la revendication 19 ou 20, dans lequel le milieu réactionnel est constitué par le tétrahydrofuranne.

**22.** Procédé suivant l'une quelconque des revendications 18 à 21, dans lequel le composé de formule :

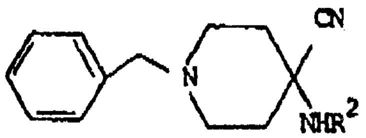

est formé par réaction de N-benzylpipéridone avec un cyanure et avec une amine de formule $R^2NH_2$.

**23.** Procédé de préparation d'une composition analgésique ou anesthésique, comprenant le mélange d'un composé suivant l'une quelconque des revendications 1 à 6 à un support pharmaceutiquement acceptable.